# EUROPEAN PATENT APPLICATION

(11) **EP 3 670 517 A1**
(43) Date of publication of application: **24.06.2020**
(21) Application number: 18215079.7
(22) Date of filing: 21.12.2018
(51) Int. Cl.: C07D 495/04, C09K 11/06, H01L 51/00, H05B 33/14

(54) **POLYCYCLIC COMPOUND, COMPOSITION AND AN ORGANIC ELECTROLUMINESCENCE DEVICE COMPRISING THE POLYCYCLIC COMPOUND OR THE COMPOSITION**

(71) Applicant: Idemitsu Kosan Co., Ltd., Chiyoda-ku, Tokyo 100-8321 (JP)
(72) Inventor: Groarke, Michelle, 4102 Binningen (BL) (CH); Mizuki, Yumiko, 4054 Basel (CH); Wolleb, Heinz, 4232 Fehren (CH); Boufflet, Pierre, 4055 Basel (CH); Kawamura, Masahiro, 4057 Basel (CH)
(74) Representative: Hollah, Dorothee

(57) **Abstract**

Specific polycyclic compounds of the general formula (Ia) or (Ib) and compositions comprising said polycyclic compounds, and a process for the preparation of the specific polycyclic compounds of the general formula (Ia) or (lb), a material for an organic electroluminescence device comprising said compounds or said compositions, an organic electroluminescence device comprising said compounds or said compositions, an electronic equipment comprising said organic electroluminescence device, and the use of compounds according to general formula (Ia) or (Ib) or the compositions comprising said polycyclic compounds in an organic electroluminescence device.

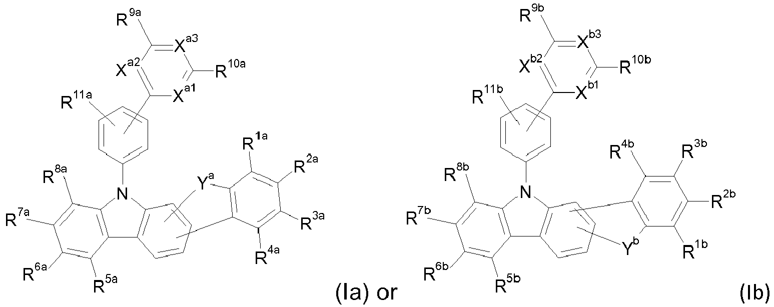

## Description

The present invention relates to specific polycyclic compounds and compositions comprising said polycyclic compounds and to organic electroluminescence devices comprising the same.

KR 2017-0049201 relates to heterocyclic compounds represented by the following formula (1) and organic light emitting devices using the same

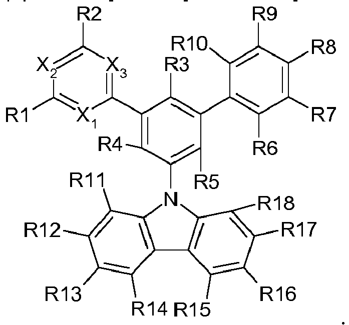

In the compounds of formula (1), at least one of R6 to R10 is -CN.

US 2017/0117488 A1 relates to an organic electroluminescence device comprising at least one light-emitting layer between an anode and a cathode, wherein the light-emitting layer comprises a host and a phosphorescent dopant; the host consists of multi-component host compounds; at least a first host compound of the multi-component host compounds is a specific biscarbazole derivative containing an aryl group, and a second host compound is a specific carbazole derivative including a nitrogen-containing heteroaryl group.

However, the specific structure and substitution pattern of polycyclic compounds has a significant impact on the performance of the polycyclic compounds in organic electronic devices.

Therefore, notwithstanding the developments described above, there remains a need for organic electroluminescence devices comprising new materials, especially host (= matrix) materials, charge blocker materials, e.g. hole blocker materials and/or charge transport materials, e.g. electron transport materials, to provide improved performance of electroluminescence devices.

Accordingly, it is an object of the present invention, with respect to the aforementioned related art, to provide further materials suitable for use in organic electroluminescence devices and further applications in organic electronics. More particularly, it should be possible to provide charge transport materials, e.g. electron transport materials, and/or charge blocker materials, e.g. hole blocker materials, and/or host (= matrix) materials for use in organic electroluminescence devices. The materials should be suitable especially for organic electroluminescence devices which comprise at least one emitter, which is a phosphorescence emitter and/or a fluorescence emitter, preferably a phosphorescence emitter, for example at least one green emitter - or - in a further embodiment - preferably a fluorescent emitter, for example at least one blue emitter.

Furthermore, the materials should be suitable for providing organic electroluminescence devices which ensure good performance of the organic electroluminescence devices, especially a long lifetime and/or low driving voltage.

Said object is solved by a polycyclic compound represented by formula (Ia) or (Ib): 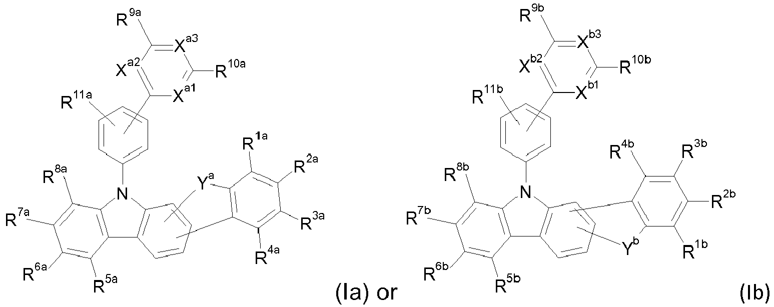 wherein
R^{1a}, R^{2a}, R^{3a}, R^{4a}, R^{5a}, R^{6a}, R^{7a}, R^{8a}, R^{1b}, R^{2b}, R^{3b}, R^{4b}, R^{5b}, R^{6b}, R^{7b} and R^{8b} each independently represents hydrogen, a halogen atom, a substituted or unsubstituted alkyl group having 1 to 25 carbon atoms, a substituted or unsubstituted alkenyl group having 2 to 25 carbon atoms, a substituted or unsubstituted alkynyl group having 2 to 25 carbon atoms, a substituted or unsubstituted cycloalkyl group having 3 to 25 carbon atoms, a substituted or unsubstituted alkoxy group having 1 to 25 carbon atoms, a substituted or unsubstituted aryl group having 6 to 18 carbon atoms, a substituted or unsubstituted heteroaryl group having 5 to 18 ring atoms, a substituted or unsubstituted aryloxy group having 6 to 24 carbon atoms, a substituted or unsubstituted alkylthio group having 1 to 25 carbon atoms, a substituted or unsubstituted arylthio group having 6 to 24 carbon atoms, an alkyl and/or aryl substituted silyl group, an alkyl or aryl substituted carbonyl group, or a substituted phosphoryl group; CN;
R^{9a}, R^{10a}, R^{9b} and R^{10b} each independently represents a substituted or unsubstituted aryl group having 6 to 18 carbon atoms or a substituted or unsubstituted heteroaryl group having 5 to 18 ring atoms;
R^{11a} and R^{11b} each independently represents an unsubstituted aryl group having 6 to 18 carbon atoms or an unsubstituted heteroaryl group having 5 to 18 ring atoms;
X^{a1}, X^{a2}, X^{a3}, X^{b1}, X^{b2} and X^{b3} each independently represents N or CR¹², wherein at least two of X^{a1}, X^{a2} and X^{a3} and at least two of X^{b1}, X^{b2} and X^{b3} are N;
R¹² represents in each case independently hydrogen, a substituted or unsubstituted aryl group having 6 to 18 carbon atoms or a substituted or unsubstituted heteroaryl group having 5 to 18 ring atoms;
Y^{a} and Y^{b} each independently represents S or O.

The specific polycyclic compounds of the present invention according to formulae (Ia) and (Ib) comprising an aryl linking group substituted by an unsubstituted aryl or heteroaryl group may be used as a material, especially host, charge transport or charge blocking material, that is highly suitable in organic electroluminescence devices. Moreover, thermally stable compounds are provided, especially resulting in organic electroluminescence devices having long lifetimes and low driving voltages.

The compounds of the present invention may also be used in further organic electronic devices than organic electroluminescence devices such as electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors and dye lasers.

Accordingly, a further subject of the present invention is directed to an organic electronic device, comprising a compound according to the present invention. The organic electronic device is preferably an organic electroluminescence device (EL device). The term organic EL device (organic electroluminescence device) is used interchangeable with the term organic light-emitting diode (OLED) in the present application.

The compounds of formulae (Ia) and (Ib) can in principal be used in any layer of an EL device, but are preferably used as host, charge transport, especially electron transport, and/or charge blocking, especially hole blocking, material. Particularly, the compounds of formulae (Ia) and (Ib) are used as host material, hole blocking material and/or electron transport material for phosphorescence or fluorescence emitters. More preferably, the compounds of formulae (Ia) and (Ib) are used as host material for phosphorescence or fluorescence emitters. Most preferably, the compounds of formulae (Ia) and (Ib) are used as host material for phosphorescence emitters. Further most preferably, the compounds of formulae (Ia) and (Ib) are used as host material for phosphorescence emitters together with a further host material.

Hence, a further subject of the present invention is directed to a material for an organic electroluminescence device comprising at least one compound of formula (Ia) or (Ib) according to the present invention.

A further subject of the present invention is directed to an organic electroluminescence device which comprises an organic thin film layer between a cathode and an anode, wherein the organic thin film layer comprises one or more layers and comprises a light emitting layer, and at least one layer of the organic thin film layer comprises at least one compound of formula (Ia) or (Ib) according to the present invention.

A further subject of the present invention is directed to an electronic equipment comprising the organic electroluminescence device according the present invention.

A further subject of the present invention is directed to the use of a compound of formula (Ia) or (Ib) according to the present invention in an organic electroluminescence device.

A further subject of the present invention is directed to an emitting layer, comprising a compound of formula (Ia) or (Ib) according to the present invention. In said embodiment the compound of formula (Ia) or (Ib) is preferably used as host material or as co-host material together with one or more, preferably one, further host materials. More preferably, a combination of a compound of formula (Ia) or (Ib) as host material or as co-host material together with a phosphorescent emitter is used.

A further subject of the present invention is directed to an electron transporting layer comprising a compound of formula (Ia) or (Ib) according to the present invention. Preferably, the electron transporting layer is provided between the cathode and the light emitting layer of an EL device such as an OLED.

A further subject of the present invention is directed to a hole blocking layer comprising a compound of formula (Ia) or (Ib) according to the present invention. Preferably, the hole blocking layer is provided between the electron transporting layer and the light emitting layer of an EL device such as an OLED.

The terms aromatic hydrocarbon group having a ring structure formed of 6 to 30 carbon atoms, aryl group having 6 to 24 carbon atoms, aryl group having 6 to 18 carbon atoms, heterocyclic group having a ring structure formed of 5 to 30 atoms, heterocyclic group having 4 to 30 carbon atoms, heteroaryl group having 5 to 18 ring atoms, alkyl group having 1 to 25 carbon atoms, alkenyl group having 2 to 25 carbon atoms, alkynyl group having 2 to 25 carbon atoms, cycloalkyl group having 3 to 25 carbon atoms, alkoxy group having 1 to 25 carbon atoms, alkylthio group having 1 to 25 carbon atoms, aryloxy group having 6 to 24 carbon atoms, arylthio group having 6 to 24 carbon atoms, alkyl and/or aryl substituted silyl group, alkyl and/or aryl substituted carbonyl group, phosphoryl group and halogen
are known in the art and generally have the following meaning, if said groups are not further specified in specific embodiments mentioned below:
The terms aralkyl group having 7 to 24 carbon atoms, alkylene group having 1 to 30 carbon atoms, cycloalkylene group having a ring structure formed of 3 to 20 carbon atoms, divalent silyl group having 2 to 20 carbon atoms, divalent aromatic hydrocarbon group having a ring structure formed of 6 to 30 carbon atoms, divalent heterocyclic group 5 to 30 ring atoms, alkyl and/or aryl substituted silyl group, alkyl and/or aryl substituted carbonyl group, halogen atom, alkoxy group having 1 to 25 carbon atoms, haloalkyl group having 1 to 25 carbon atoms, haloalkoxy group having 1 to 25 carbon atoms, aryloxy group having 6 to 24 ring carbon atoms, alkylthio group having 1 to 25 carbon atoms, arylthio group having 6 to 24 ring carbon atoms, substituted phosphoryl group, alkylamino group having 1 to 25 carbon atoms, arylamino group having 6 to 24 carbon atoms, alkyl or aryl substituted carbonyl group, carboxyalkyl group having 1 to 25 carbon atoms, carboxamidalkyl group having 1 to 25 carbon atoms, carboxyaryl group having 6 to 24 carbon atoms, carboxamidaryl group having 6 to 24 carbon atoms,
are known in the art and generally have the following meaning, if said groups are not further specified in specific embodiments mentioned below:
The aromatic hydrocarbon group having a ring structure formed of 6 to 30 carbon atoms (aromatic hydrocarbon group having 6 to 30 ring carbon atoms), preferably 6 to 24 ring carbon atoms or the substituted or unsubstituted aryl group having 6 to 24 carbon atoms, may be a non-condensed aromatic hydrocarbon group or a condensed aromatic hydrocarbon group, preferably, the aromatic hydrocarbon group having 6 to 24 ring carbon atoms is an aryl group having 6 to 24 carbon atoms. Specific examples thereof include phenyl group, naphthyl group, phenanthryl group, biphenyl group, terphenyl group, quaterphenyl group, fluoranthenyl group, triphenylenyl group, phenanthrenyl group, fluorenyl group, anthracenyl, chrysenyl, spirofluorenyl group, 9,9-diphenylfluorenyl group, 9,9'-spirobi[9H-fluorene]-2-yl group, 9,9-dimethylfluorenyl group, benzo[c]phenanthrenyl group, benzo[a]triphenylenyl group, naphtho[1,2-c]phenanthrenyl group, naphtho[1,2-a]triphenylenyl group, dibenzo[a,c]triphenylenyl group, benzo[a]fluoranthenyl group, benzo[j]fluoranthenyl group, benzo[k]fluoranthenyl group and benzo[b]fluoranthenyl group, with phenyl group, naphthyl group, biphenyl group, terphenyl group, phenanthryl group, triphenylenyl group, fluorenyl group, spirobifluorenyl group, and fluoranthenyl group being preferred, and phenyl group, 1-naphthyl group, 2-naphthyl group, biphenyl-2-yl group, biphenyl-3-yl group, biphenyl-4-yl group, phenanthrene-9-yl group, phenanthrene-3-yl group, phenanthrene-2-yl group, triphenylene-2-yl group, 9,9-dimethylfluorene-2-yl group, fluoranthene-3-yl group, fluoranthene-2-yl group, fluoranthene-8-yl group being more preferred.

The substituted or unsubstituted aryl group having 6 to 18 carbon atoms, may be a non-condensed aryl group or a condensed aryl group. Specific examples thereof include phenyl group, naphthyl group, phenanthryl group, biphenyl group, terphenyl group, fluoranthenyl group, triphenylenyl group, phenanthrenyl group, fluorenyl group, anthracenyl, chrysenyl, spirofluorenyl group, with phenyl group, naphthyl group, biphenyl group, terphenyl group, phenanthryl group, triphenylenyl group, fluorenyl group, spirobifluorenyl group, and fluoranthenyl group being preferred, and phenyl group, 1-naphthyl group, 2-naphthyl group, biphenyl-2-yl group, biphenyl-3-yl group, biphenyl-4-yl group, phenanthrene-9-yl group, phenanthrene-3-yl group, phenanthrene-2-yl group, triphenylene-2-yl group, 9,9-dimethylfluorene-2-yl group, fluoranthene-3-yl group, fluoranthene-2-yl group, fluoranthene-8-yl group being more preferred.

The heteroaryl group having 5 to 18 ring atoms may be a non-condensed heterocyclic group or a condensed heterocyclic group. Specific examples thereof include the residues of pyrrole ring, isoindole ring, benzofuran ring, isobenzofuran ring, benzothiophene, dibenzothiophene ring, isoquinoline ring, quinoxaline ring, quinazoline, phenanthridine ring, phenanthroline ring, pyridine ring, pyrazine ring, pyrimidine ring, pyridazine ring, indole ring, quinoline ring, acridine ring, carbazole ring, furan ring, thiophene ring, benzoxazole ring, benzothiazole ring, benzimidazole ring, dibenzofuran ring, with the residues of dibenzofuran ring, carbazole ring, and dibenzothiophene ring being preferred, and the residues of dibenzofuran-1-yl group, dibenzofuran-3-yl group, dibenzofuran-2-yl group, dibenzofuran-4-yl group, 9-phenylcarbazole-3-yl group, 9-phenylcarbazole-2-yl group, 9-phenylcarbazole-4-yl group, dibenzothiophene-2-yl group, and dibenzothiophene-4-yl, dibenzothiophene-1-yl group, and dibenzothiophene-3-yl group being more preferred.

The heterocyclic group having a ring structure formed of 5 to 30 atoms (heterocyclic group having 5 to 30 ring atoms), preferably 5 to 18 ring atoms, may be a non-condensed heterocyclic group or a condensed heterocyclic group. Specific examples and preferred examples are the same groups as mentioned above concerning the heterocyclic group having a ring structure formed of 4 to 30 carbon atoms. Further specific examples are: triazine ring, oxazole ring, oxadiazole ring, thiazole ring, thiadiazole ring, triazole ring, and imidazole ring.

Examples of the alkyl group having 1 to 25 carbon atoms include methyl group, ethyl group, n-propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neopentyl group, 1-methylpentyl group, with methyl group, ethyl group, n-propyl group, isopropyl group, n-butyl group, s-butyl group, isobutyl group, t-butyl group being preferred. Preferred are alkyl groups having 1 to 8 carbon atoms. Suitable examples for alkyl groups having 1 to 8 carbon atoms are mentioned before.
Examples of the alkenyl group having 2 to 25 carbon atoms include those disclosed as alkyl groups having 2 to 25 carbon atoms but comprising at least one double bond, preferably one, or where possible, two or three double bonds.

Examples of the alkynyl group having 2 to 25 carbon atoms include those disclosed as alkyl groups having 2 to 25 carbon atoms but comprising at least one triple bond, preferably one, or where possible, two or three triple bonds.

Examples of the cycloalkyl group having 3 to 25 carbon atoms include cyclopropyl group, cyclobutyl group, cyclopentyl group, cyclohexyl group, cyclooctyl group, and adamantyl group, with cyclopentyl group, and cyclohexyl group being preferred. Preferred are cycloalkyl groups having 3 to 6 carbon atoms. Suitable examples for cycloalkyl groups having 3 to 6 carbon atoms are mentioned before.

Examples of the alkylene group (i.e. alkane-diyl group) having 1 to 30 carbon atoms represented include methylene group, ethylene group, n-propylene group, isopropylene group, n-butylene group, s-butylene group, isobutylene group, t-butylene group, n-pentylene group, n-hexylene group, n-heptylene group, n-octylene group, n-nonylene group, n-decylene group, n-undecylene group, n-dodecylene group, n-tridecylene group, n-tetradecylene group, n-pentadecylene group, n-hexadecylene group, n-heptadecylene group, n-octadecylene group, neopentylene group, 1-methylpentylene group, with methylene group, ethylene group, n-propylene group, isopropylene group, n-butylene group, s-butylene group, isobutylene group, t-butylene group being preferred.

Examples of the cycloalkylene group (i.e. cycloalkane-diyl group) having 3 to 20 carbon atoms include cyclopropylene group, cyclobutylene group, cyclopentylene group, cyclohexylene group, cyclooctylene group, and adamantylene group, with cyclopentylene group, and cyclohexylene group being preferred.

Examples of the substituted divalent silyl group having 2 to 20 carbon atoms include divalent dimethylsilyl group, divalent diethylsilyl group, divalent dibutylsilyl group, divalent methylethylsilyl group, divalent t-butylmethylsilyl group, divalent vinylmethylsilyl group, divalent propylmethylsilyl group, divalent methylisopropylsilyl group, divalent methylpropylsilyl group, divalent methylbutylsilyl group, divalent methyltertiarybutylsilyl group, divalent ethylisopropylsilyl group, divalent phenylmethylsilyl group, divalent phenylmethylsilyl group, divalent phenyltertiarybutylsilyl group, and divalent diphenylsilyl group, with divalent dimethylsilyl group, divalent diethylsilyl group, divalent t-butylmethylsilyl group, divalent vinylmethylsilyl group, and divalent propylmethylsilyl group being preferred.

The divalent aromatic hydrocarbon group having 6 to 30 ring carbon atoms may be a non-condensed divalent aromatic hydrocarbon group or a condensed divalent aromatic hydrocarbon group. Specific examples thereof include phenylene group, naphthylene group, phenanthrylene group, biphenyl-diyl group, terphenyl-diyl group, quaterphenyl-diyl group, fluoranthen-diyl group, triphenylenylene-diyl group, phenanthrene-diyl group, fluorene-diyl group, spirofluorene-diyl group, 9,9-diphenylfluorene-diyl group, 9,9'-spirobi[9H-fluorene]-2-diyl group, 9,9-dimethylfluorene-diyl group, benzo[c]phenanthrene-diyl group, benzo[a]triphenylene-diyl group, naphtho[1,2-c]phenanthrene-diyl group, naphtho[1,2-a]triphenylenylene-diyl group, dibenzo[a,c]triphenylenylene-diyl group, benzo[a]fluoranthene-diyl group, benzo[j]fluoranthene-diyl group, benzo[k]fluoranthene-diyl group, and benzo[b]fluoranthene-diyl group, with phenylene group, naphthylene group, biphenyl-diyl group, terphenyl-diyl group, phenanthryl-diyl group, triphenylenylen-diyl group, fluorene-diyl group, spirobifluorene-diyl group, and fluoranthene-diyl group being preferred, and 1,2-phenylene group, 1,3-phenylene group, 1,4-phenylene group, 1,4-naphthylene group, 1,8-naphthylene group, 2,6-naphthylene group, 2,7-naphthylene group, biphenyl-2,2'-diyl group, biphenyl-2,3'-diyl group, biphenyl-2,4'-diyl group, biphenyl-2,5'-diyl group, biphenyl-2,6'-diyl group, biphenyl-3,3'-diyl group, biphenyl-3,4'-diyl group, biphenyl-3,5'-diyl group, biphenyl-3,6'-diyl group, biphenyl-4,4'-diyl group, biphenyl-4,5'-diyl group, biphenyl-4,6'-diyl group, biphenyl-5,5'-diyl group, biphenyl-5,6'-diyl group, biphenyl-6,6'-diyl group, phenanthrene-9,10-diyl group, phenanthrene-2,3-diyl group, phenanthrene-2,7-diyl group, phenanthrene-2,8-diyl group, phenanthrene-2,6-diyl group, phenanthrene-2,9-diyl group, phenanthrene-2,10-diyl group, phenanthrene-3,9-diyl group, phenanthrene-3,10-diyl group, triphenylene-2,3-diyl group, triphenylene-2,5-diyl group, triphenylene-2,6-diyl group, triphenylene-2,7-diyl group, triphenylene-2,8-diyl group, 9,9-dimethylfluorene-2,7-diyl group, 9,9-dimethylfluorene-3,7-diyl group, 9,9-dimethylfluorene-1,4-diyl group, fluoranthene-3,9-diyl group, fluoranthene-3,8-diyl group, fluoranthene-3,4-diyl group, fluoranthene-3,5-diyl group, fluoranthene-3,6-diyl group, fluoranthene-2,9-diyl group, fluoranthene-2,8-diyl group, fluoranthene-2,4-diyl group, fluoranthene-2,5-diyl group, fluoranthene-2,6-diyl group, fluoranthene-1,9-diyl group, fluoranthene-1,8-diyl group, fluoranthene-1,4-diyl group, fluoranthene-1,5-diyl group, fluoranthene-1,6-diyl group being more preferred.

The divalent heterocyclic group having 5 to 30 ring atoms, preferably 4 to 30 carbon atoms, may be a non-condensed heterocyclic group or a condensed heterocyclic group. Specific examples thereof include the divalent residues of pyrrole ring, isoindole ring, benzofuran ring, isobenzofuran ring, dibenzothiophene ring, isoquinoline ring, quinoxaline ring, quinazoline ring, phenanthridine ring, phenanthroline ring, pyridine ring, pyrazine ring, pyrimidine ring, pyridazine ring, triazine ring, indole ring, quinoline ring, acridine ring, pyrrolidine ring, dioxane ring, piperidine ring, morpholine ring, piperazine ring, carbazole ring, furan ring, thiophene ring, benzoxazole ring, benzothiazole ring, benzimidazole ring, pyran ring, dibenzofuran ring, and benzo[c]dibenzofuran ring, with the divalent residues of dibenzofuran ring, carbazole ring, dibenzothiophene ring being preferred, and the dibenzofuran-diyl group, 9-phenylcarbazole-diyl group and dibenzothiophene-diyl group being more preferred. Examples of aryl and/or alkyl substituted silyl groups including alkylsilyl groups having 1 to 10 carbon atoms, preferably 1 to 5 carbon atoms, including trimethylsilyl group, triethylsilyl group, tributylsilyl group, dimethylethylsilyl group, t-butyldimethylsilyl group, vinyldimethylsilyl group, propyldimethylsilyl group, dimethylisopropylsilyl group, dimethylpropylsilyl group, dimethylbutylsilyl group, dimethyltertiarybutylsilyl group, diethylisopropylsilyl group, and arylsilyl groups having 6 to 30 carbon atoms, preferably 6 to 18 carbon atoms, including phenyldimethylsilyl group, diphenylmethylsilyl group, diphenyltertiarybutylsilyl group, and triphenylsilyl group, with trimethylsilyl group, triethylsilyl group, t-butyldimethylsilyl group, vinyldimethylsilyl group, and propyldimethylsilyl group being preferred.

Examples of halogen atoms include fluorine, chlorine, bromine, and iodine, with fluorine being preferred.

Examples of an alkoxy group having 1 to 25 carbon atoms, preferably 1 to 8 carbon atoms, include those having an alkyl portion selected from the alkyl groups mentioned above.

Examples of an aryloxy group having 6 to 24 ring carbon atoms include those having an aryl portion selected from the aromatic hydrocarbon groups mentioned above.

Examples of an alkylthio group having 1 to 25 carbon atoms include those having an alkyl portion selected from the alkyl groups mentioned above.

Examples of an arylthio group having 6 to 24 ring carbon atoms include those having an aryl portion selected from the aromatic hydrocarbon groups mentioned above.

Examples of substituted phosphoryl groups are di-substituted phosphoryl groups having a substituent selected from the group consisting of a substituted or unsubstituted alkyl group having 1 to 25 carbon atoms and a substituted or unsubstituted aromatic hydrocarbon group having 6 to 24 ring carbon atoms. A preferred phosphoryl group is a diphenylphosphin oxide group.

Examples of alkyl or aryl substituted carbonyl groups include those having an alkyl portion selected from the alkyl groups mentioned above and/or having an aryl portion selected from the aromatic hydrocarbon groups mentioned above.

Examples of a haloalkyl group having 1 to 25 carbon atoms include the alkyl groups mentioned above wherein the hydrogen atoms thereof are partly or entirely substituted by halogen atoms.

Examples of a haloalkoxy group having 1 to 25 carbon atoms include the alkoxyl group mentioned above wherein the hydrogen atoms thereof are partly or entirely substituted by halogen atoms.

Examples of an alkylamino group having 1 to 25 ring carbon atoms include those having an alkyl portion selected from the alkyl groups mentioned above.

Examples of an arylamino group having 6 to 24 ring carbon atoms include those having an aryl portion selected from the aromatic hydrocarbon groups mentioned above.

Examples of a carboxyalkyl group having 1 to 25 carbon atoms, preferably 1 to 5 carbon atoms, include those having an alkyl portion selected from the alkyl groups mentioned above.

Examples of a carboxamidalkyl group having 1 to 25 carbon atoms, preferably 1 to 5 carbon atoms include those having an alkyl portion selected from the alkyl groups mentioned above.

Examples of a carboxyaryl group having 6 to 24 carbon atoms, preferably 6 to 18 carbon atoms, include those having an aryl portion selected from the aromatic hydrocarbon groups mentioned above.

Examples of a carboxamidaryl group having 6 to 24 carbon atoms, preferably 6 to 18 carbon atoms, include those having an aryl portion selected from the aromatic hydrocarbon groups mentioned above.

Examples of the optional substituent(s) indicated by "substituted or unsubstituted" and "may be substituted" referred to above or hereinafter include a halogen atom (fluorine, chlorine, bromine, iodine), a cyano group, an alkyl group having 1 to 25, preferably 1 to 6 carbon atoms, a cycloalkyl group having 3 to 25, preferably 5 to 12 carbon atoms, an alkoxyl group having 1 to 25, preferably 1 to 5 carbon atoms, a haloalkyl group having 1 to 25, preferably 1 to 5 carbon atoms, a haloalkoxyl group having 1 to 25, preferably 1 to 5 carbon atoms, an alkylamino group having 1 to 25 carbon atoms, preferably 1 to 5 carbon atoms, a carboxyalkyl group having 1 to 25 carbon atoms, preferably 1 to 5 carbon atoms, a carboxamidalkyl group having 1 to 25 carbon atoms, preferably 1 to 5 carbon atoms, a silyl group, an aromatic hydrocarbon group having 6 to 24 ring carbon atoms, preferably 6 to 18 ring carbon atoms, an aryloxy group having 6 to 24, preferably 6 to 18 ring carbon atoms, an aralkyl group having 7 to 24, preferably 7 to 20 carbon atoms, an alkylthio group having 1 to 25, preferably 1 to 5 carbon atoms, an arylthio group having 6 to 24, preferably 6 to 18 ring carbon atoms, an arylamino group having 6 to 30 carbon atoms, preferably 6 to 18 carbon atoms, a carboxyaryl group having 6 to 24 carbon atoms, preferably 6 to 18 carbon atoms, a carboxamidaryl group having 6 to 24 carbon atoms, preferably 6 to 18 carbon atoms, and an heterocyclic group having 5 to 24 ring atoms, preferably 5 to 18 ring atoms.

The optional substituent is preferably a fluorine atom, a cyano group, an alkyl group having 1 to 25 carbon atoms, an aromatic hydrocarbon group having 6 to 24 ring carbon atoms, preferably 6 to 18 ring carbon atoms, and an heterocyclic group having 5 to 24 ring atoms, preferably 5 to 18 ring atoms; more preferably a cyano group, a phenyl group, a naphthyl group, a biphenyl group, a terphenyl group, a phenanthryl group, a triphenylenyl group, a fluorenyl group, a spirobifluorenyl group, a fluoranthenyl group, a residue based on a dibenzofuran ring, a residue based on a carbazole ring, and a residue based on a dibenzothiophene ring, a methyl group, an ethyl group, an n-propyl group, an isopropyl group, an n-butyl group, an s-butyl group, an isobutyl group, a t-butyl group, a cyclopentyl group, and a cyclohexyl group.

The optional substituent mentioned above may be further substituted by one or more of the optional substituents mentioned above.

The number of the optional substituents depends on the group which is substituted by said substituent(s). Preferred are 1, 2, 3 or 4 optional substituents, more preferred are 1, 2 or 3 optional substituents, most preferred are 1 or 2 optional substituents. In a further preferred embodiment, the groups mentioned above are unsubstituted.

The "carbon number of a to b" in the expression of "substituted or unsubstituted X group having a to b carbon atoms" is the carbon number of the unsubstituted X group and does not include the carbon atom(s) of an optional substituent.

The hydrogen atom referred to herein includes isotopes different from neutron numbers, i.e., light hydrogen (protium), heavy hydrogen (deuterium) and tritium.

The term "unsubstituted" referred to by "unsubstituted or substituted" means that a hydrogen atom is not substituted by one the groups mentioned above.

An index of 0 in the definition in any formula mentioned above and below means that a hydrogen atom is present at the position defined by said index.

### R^{1a}, R^{2a}, R^{3a}, R^{4a}, R^{5a}, R^{6a}, R^{7a}, R^{8a}, R^{1b}, R^{2b}, R^{3b}, R^{4b}, R^{5b}, R^{6b}, R^{7b} and R^{8b}

R^{1a}, R^{2a}, R^{3a}, R^{4a}, R^{5a}, R^{6a}, R^{7a}, R^{8a}, R^{1b}, R^{2b}, R^{3b}, R^{4b}, R^{5b}, R^{6b}, R^{7b} and R^{8b} each independently represents hydrogen, a halogen atom, a substituted or unsubstituted alkyl group having 1 to 25 carbon atoms, a substituted or unsubstituted alkenyl group having 2 to 25 carbon atoms, a substituted or unsubstituted alkynyl group having 2 to 25 carbon atoms, a substituted or unsubstituted cycloalkyl group having 3 to 25 carbon atoms, a substituted or unsubstituted alkoxy group having 1 to 25 carbon atoms, a substituted or unsubstituted aryl group having 6 to 18 carbon atoms, a substituted or unsubstituted heteroaryl group having 5 to 18 ring atoms, a substituted or unsubstituted aryloxy group having 6 to 24 carbon atoms, a substituted or unsubstituted alkylthio group having 1 to 25 carbon atoms, a substituted or unsubstituted arylthio group having 6 to 24 carbon atoms, an alkyl and/or aryl substituted silyl group, an alkyl or aryl substituted carbonyl group, or a substituted phosphoryl group or CN.

Preferably, R^{1a}, R^{2a}, R^{3a}, R^{4a}, R^{5a}, R^{6a}, R^{7a}, R^{8a}, R^{1b}, R^{2b}, R^{3b}, R^{4b}, R^{5b}, R^{6b}, R^{7b} and R^{8b} each independently represents hydrogen, a substituted or unsubstituted phenyl group, CN, a substituted or unsubstituted biphenyl group, a substituted or unsubstituted terphenyl group, a substituted or unsubstituted triphenylenyl group, a substituted or unsubstituted naphthyl group, a substituted or unsubstituted phenanthrenyl group, a substituted or unsubstituted dibenzofuranyl group or a substituted or unsubstituted dibenzothiophenyl group.

More preferably, R^{1a}, R^{2a}, R^{3a}, R^{4a}, R^{5a}, R^{6a}, R^{7a}, R^{8a}, R^{1b}, R^{2b}, R^{3b}, R^{4b}, R^{5b}, R^{6b}, R^{7b} and R^{8b} each represents hydrogen, a substituted or unsubstituted phenyl group or CN, most preferably hydrogen or a substituted or unsubstituted phenyl group.

In a preferred embodiment of the present invention, 0, 1, 2 or 3 of the residues R^{1a}, R^{2a}, R^{3a}, R^{4a}, R^{5a}, R^{6a}, R^{7a}, R^{8a}, R^{1b}, R^{2b}, R^{3b}, R^{4b}, R^{5b}, R^{6b}, R^{7b} and R^{8b} are not hydrogen, more preferably 0, 1 or 2 of the residues R^{1a}, R^{2a}, R^{3a}, R^{4a}, R^{5a}, R^{6a}, R^{7a}, R^{8a}, R^{1b}, R^{2b}, R^{3b}, R^{4b}, R^{5b}, R^{6b}, R^{7b} and R^{8b} are not hydrogen, further more preferably 0 or 1 of the residues R^{1a}, R^{2a}, R^{3a}, R^{4a}, R^{5a}, R^{6a}, R^{7a}, R^{8a}, R^{1b}, R^{2b}, R^{3b}, R^{4b}, R^{5b}, R^{6b}, R^{7b} and R^{8b} are not hydrogen.

Most preferably, R^{1a}, R^{2a}, R^{3a}, R^{4a}, R^{5a}, R^{6a}, R^{7a}, R^{8a}, R^{1b}, R^{2b}, R^{3b}, R^{4b}, R^{5b}, R^{6b}, R^{7b} and R^{8b} are hydrogen.

### R^{9a}, R^{10a}, R^{9b} and R^{10b}

R^{9a}, R^{10a}, R^{9b} and R^{10b} each independently represents a substituted or unsubstituted aryl group having 6 to 18 carbon atoms or a substituted or unsubstituted heteroaryl group having 5 to 18 ring atoms.

Preferably, R^{9a}, R^{10a}, R^{9b} and R^{10b} each independently represents a substituted or unsubstituted phenyl group, a substituted or unsubstituted biphenyl group, a substituted or unsubstituted terphenyl group, a substituted or unsubstituted triphenylenyl group, a substituted or unsubstituted naphthyl group, a substituted or unsubstituted phenanthrenyl group, a substituted or unsubstituted dibenzofuranyl group or a substituted or unsubstituted dibenzothiophenyl group.

More preferably, R^{9a}, R^{10a}, R^{9b} and R^{10b} each independently represents an unsubstituted phenyl group, an unsubstituted biphenyl group, an unsubstituted dibenzofuranyl group or an unsubstituted dibenzothiophenyl group.

### X^{a1}, X^{a2}, X^{a3}, X^{b1}, X^{b2} and X^{b3}

X^{a1}, X^{a2}, X^{a3}, X^{b1}, X^{b2} and X^{b3} each independently represents N or CR¹², wherein at least two of X^{a1}, X^{a2} and X^{a3} and at least two of X^{b1}, X^{b2} and X^{b3} are N;

R¹² represents in each case independently hydrogen, a substituted or unsubstituted aryl group having 6 to 18 carbon atoms or a substituted or unsubstituted heteroaryl group having 5 to 18 ring atoms; preferably hydrogen, an unsubstituted aryl group having 6 to 18 carbon atoms or an unsubstituted heteroaryl group having 5 to 18 ring atoms; more preferably hydrogen or unsubstituted phenyl.

Preferably, X^{a1}, X^{a2}, X^{a3}, X^{b1}, X^{b2} and X^{b3} each represents N.

### Y^{a} and Y^{b}

Y^{a} and Y^{b} each independently represents S or O, preferably S.

### R^{11a} and R^{11b}

R^{11a} and R^{11b} each independently represents an unsubstituted aryl group having 6 to 18 carbon atoms or an unsubstituted heteroaryl group having 5 to 18 ring atoms.

The polycyclic compounds according to the present invention are characterized by a specifically substituted aryl linking group, i.e., the aryl linking group is substituted by an unsubstituted aryl group having 6 to 18 carbon atoms or an unsubstituted heteroaryl group having 5 to 18 ring atoms. It has been found by the inventors that the inventive polycyclic compounds are particularly suitable for organic electroluminescence devices.

Preferably, R^{11a} and R^{11b} each independently represents an unsubstituted phenyl group, an unsubstituted biphenyl group, an unsubstituted terphenyl group, an unsubstituted triphenylene group, a substituted or unsubstituted naphthyl group, a substituted or unsubstituted phenanthrenyl group, an unsubstituted dibenzofuranyl group or an unsubstituted dibenzothiophenyl group.

More preferably, R^{11a} and R^{11b} each independently represents an unsubstituted phenyl group, an unsubstituted biphenyl group, an unsubstituted dibenzofuranyl group or an unsubstituted dibenzothiophenyl group.

### Compounds of formula (Ia) or (Ib)

Preferably, the compounds of formula (Ia) or (Ib) are represented by the following formula (Iaa) or (Iba): 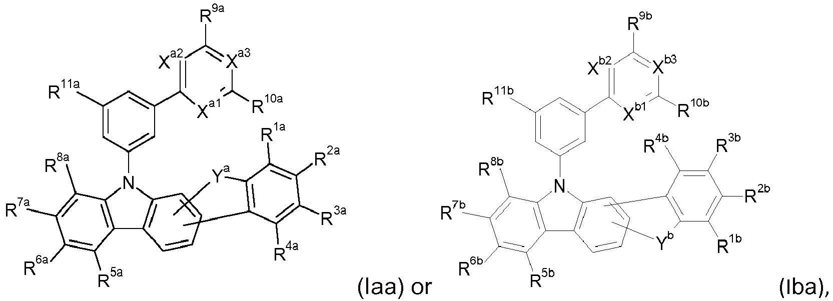 wherein the groups and residues R^{1a}, R^{2a}, R^{3a}, R^{4a}, R^{5a}, R^{6a}, R^{7a}, R^{8a}, R^{9a}, R^{10a}, R^{11a}, X^{a1}, X^{a2}, X^{a3} and Y^{a}, R^{1b}, R^{2b}, R^{3b}, R^{4b}, R^{5b}, R^{6b}, R^{7b}, R^{8b}, R^{9b}, R^{10b}, R^{11b} X^{b1}, X^{b2}, X^{b3} and Y^{b} are defined above and below.

More preferably, the compounds of formula (Ia) or (Ib) are represented by the following formula (Iaa1) or (Iba1): 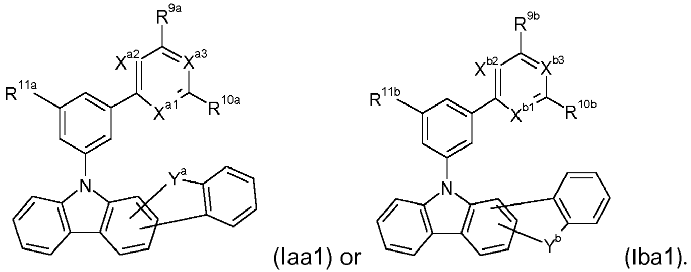 wherein the groups and residues R^{9a}, R^{10a}, R^{11a}, X^{a1}, X^{a2}, X^{a3} and Y^{a}, R^{9b}, R^{10b}, R^{11b}, X^{b1}, X^{b2}, X^{b3} and Y^{b} are defined above and below.

Further more preferably, the compounds of formula (Ia) or (Ib) are represented by the following formula (Iaa1a) or (Iba1a): 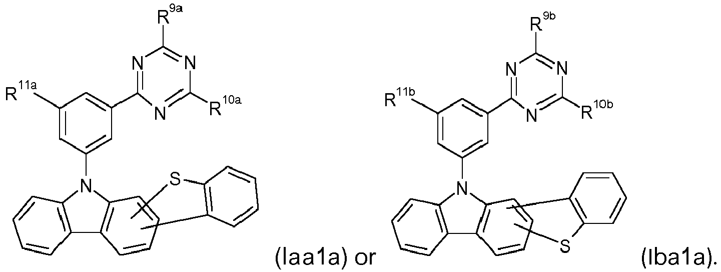 wherein the groups and residues R^{9a}, R^{10a}, R^{11a}, X^{a1}, X^{a2}, X^{a3}, R^{9b}, R^{10b}, R^{11b}, X^{b1}, X^{b2} and X^{b3} are defined above and below.

Even more preferably, the compounds of formula (Ia) or (Ib) are represented by the following formula (Iaa1a1), (Iaa1a2), (Iaa1a3) (Iba1a1), (Iba1a2) or (Iba1a3): 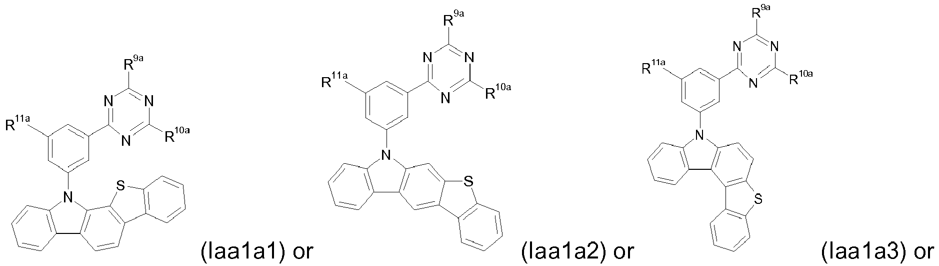 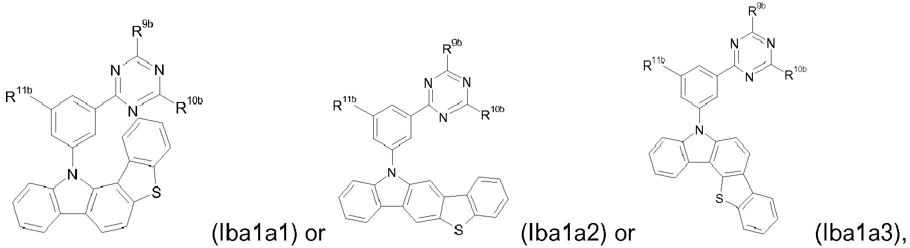 wherein the groups and residues R^{9a}, R^{10a}, R^{11a}, X^{a1}, X^{a2}, X^{a3}, R^{9b}, R^{10b}, R^{11b}, X^{b1}, X^{b2} and X^{b3} are defined above and below.

Below, examples for compounds of formulae (Ia) and (Ib) are given:

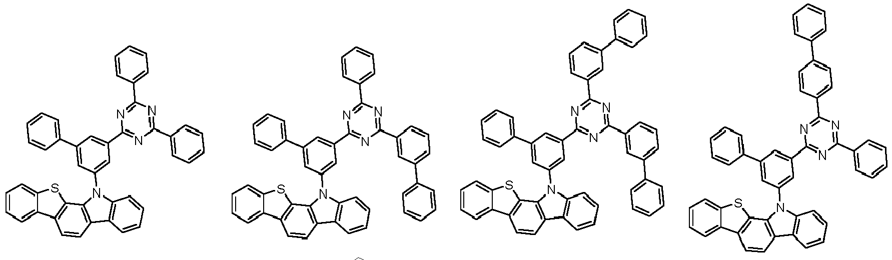

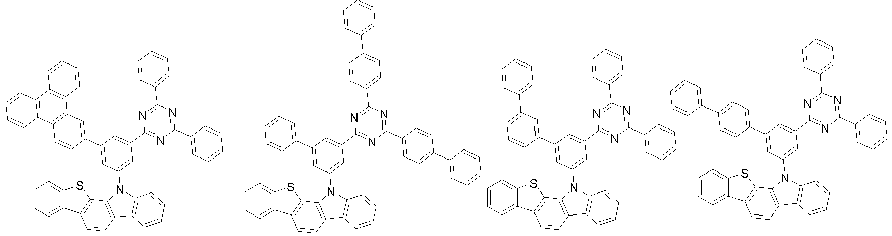

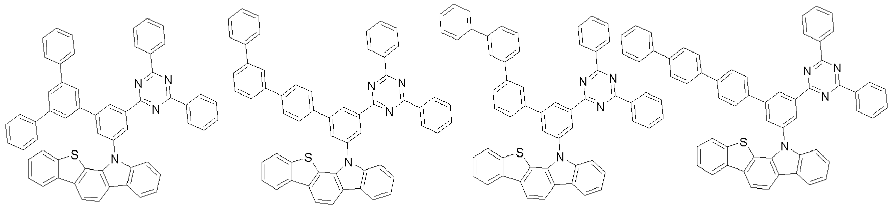

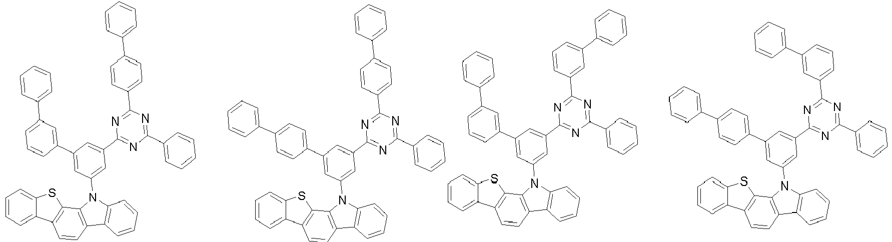

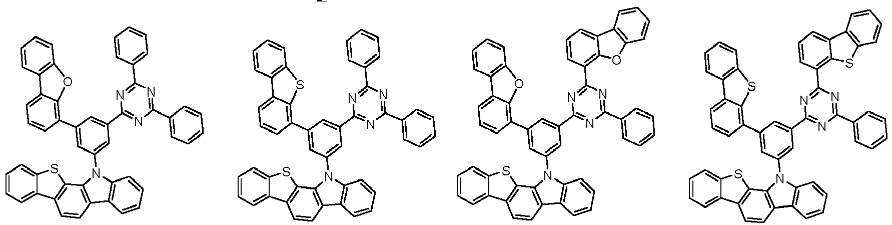

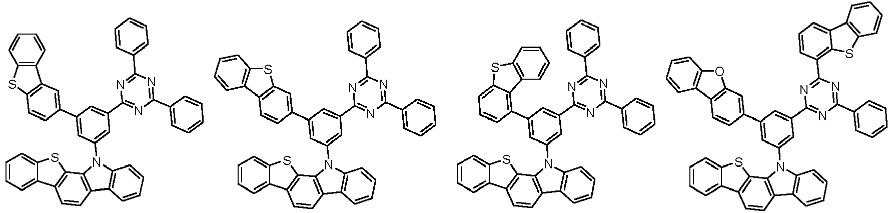

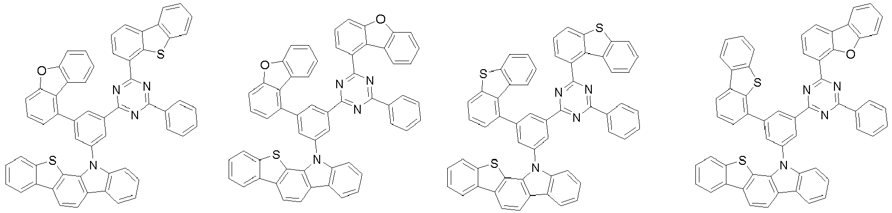

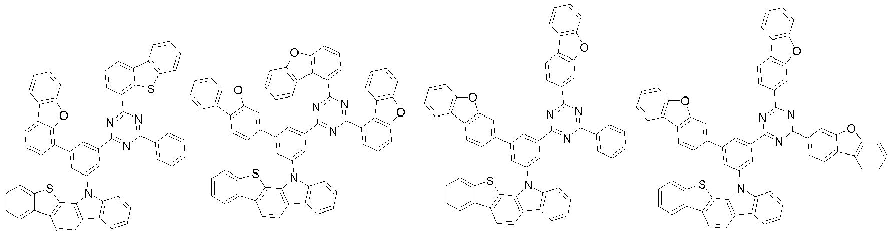

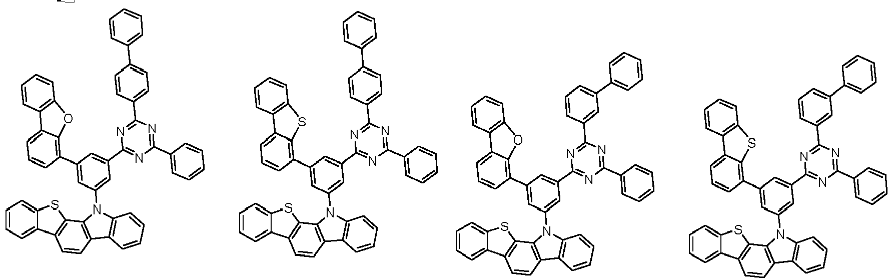

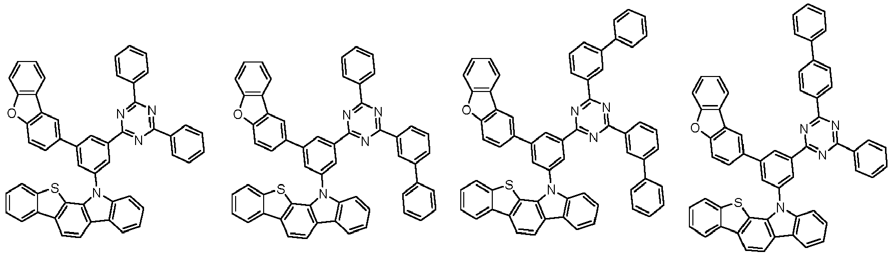

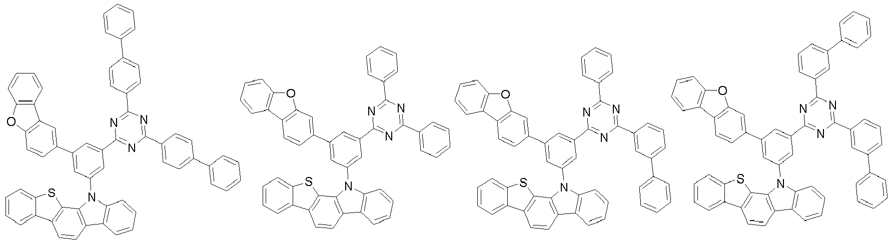

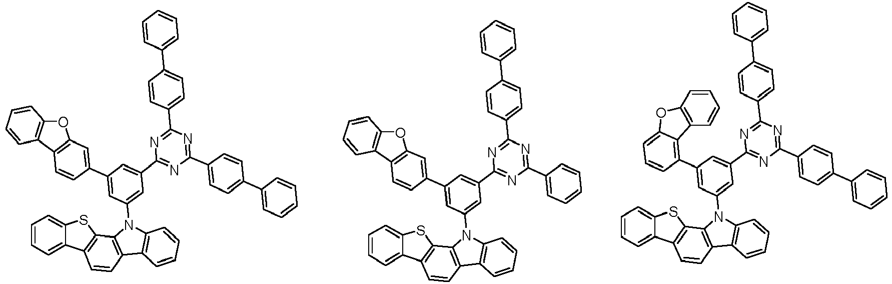

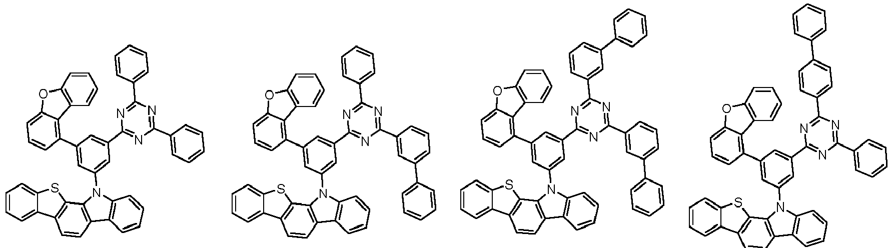

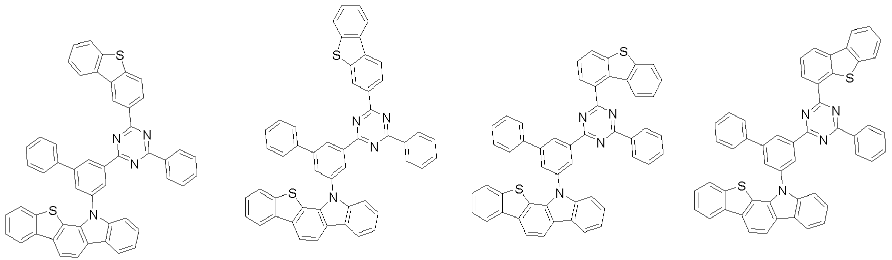

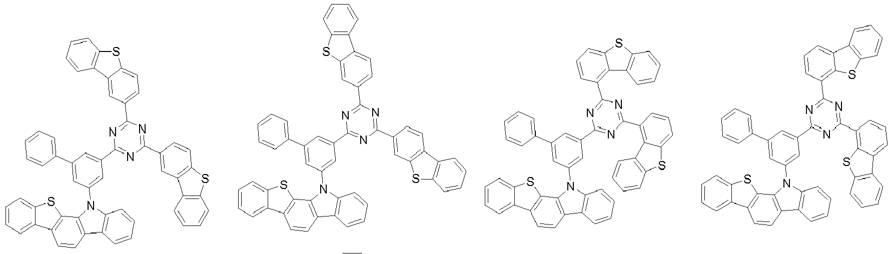

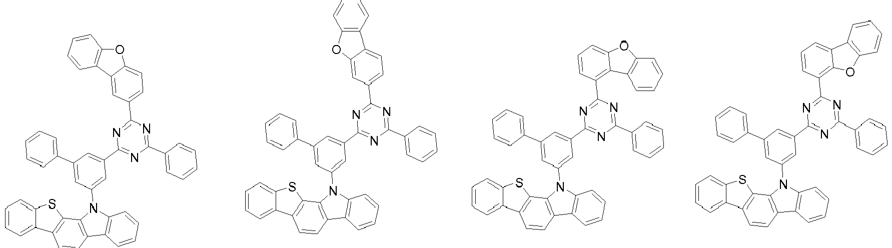

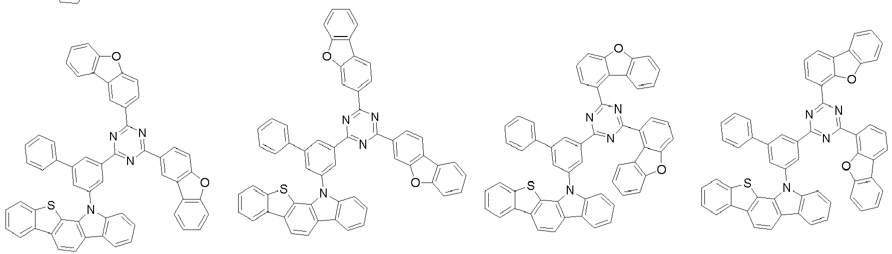

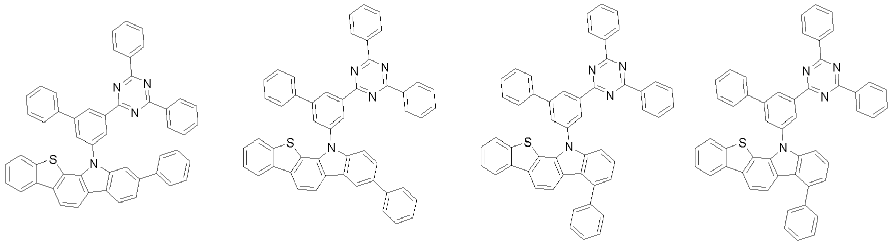

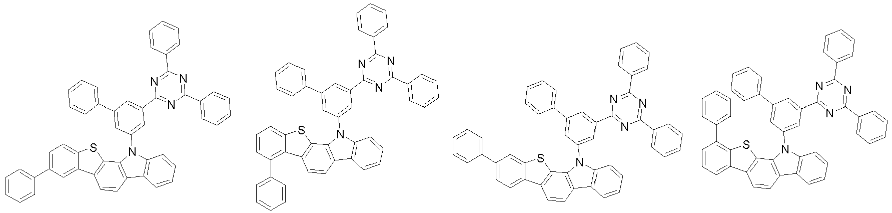

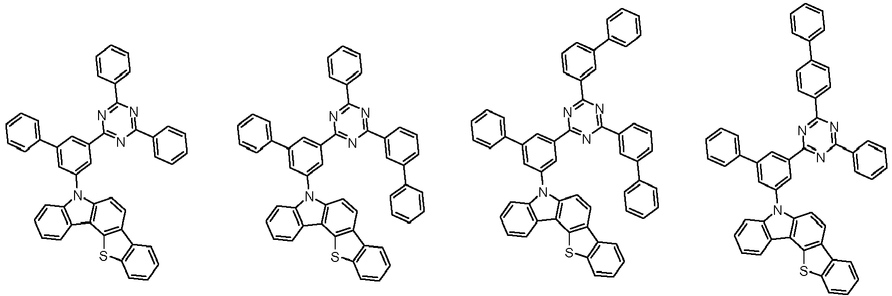

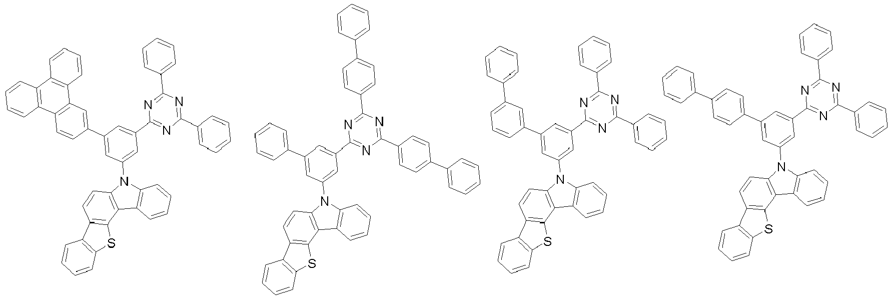

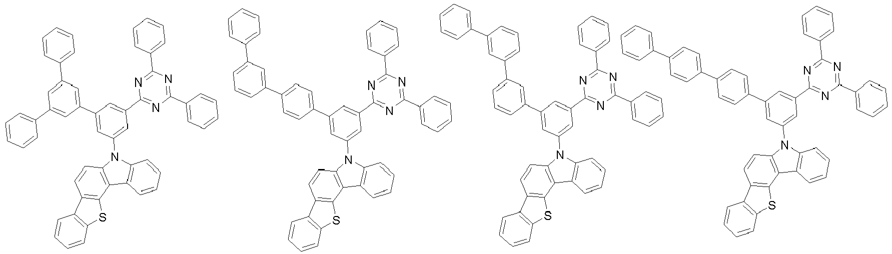

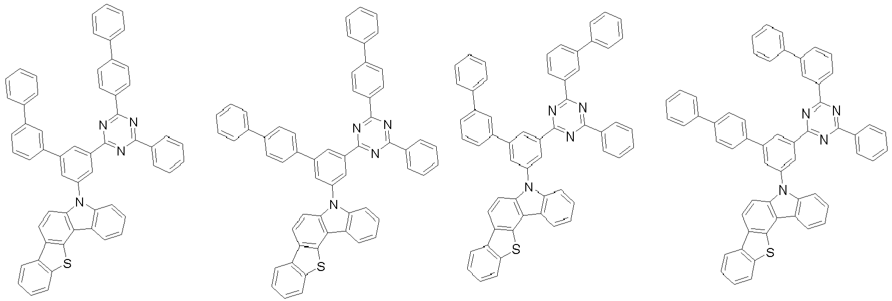

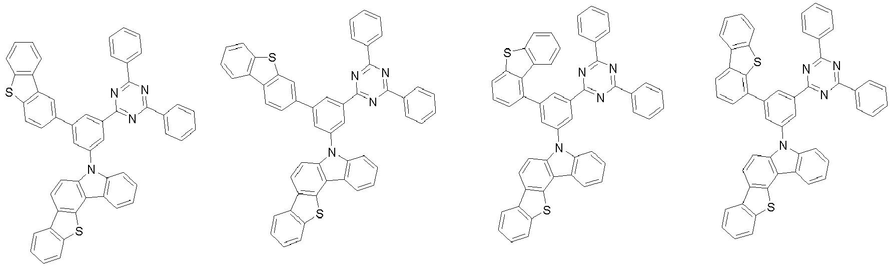

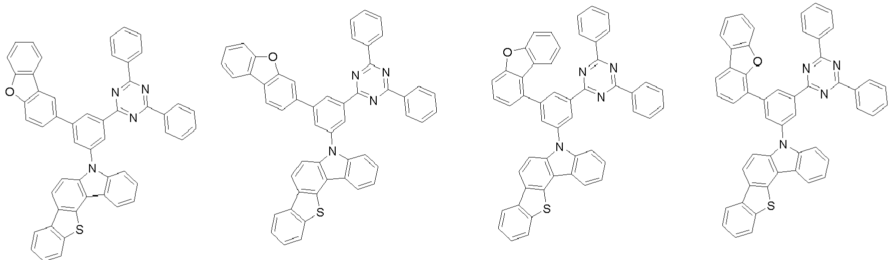

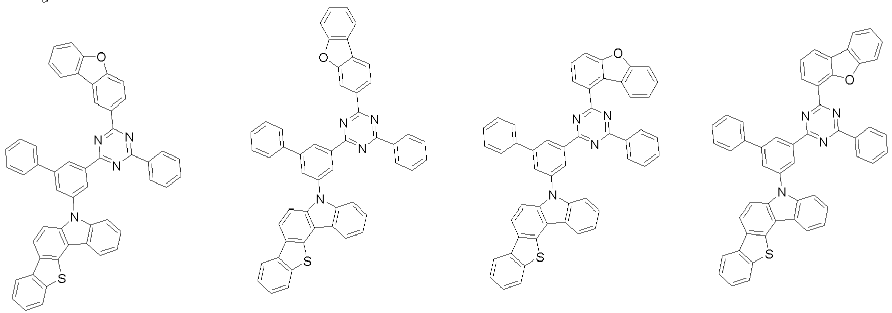

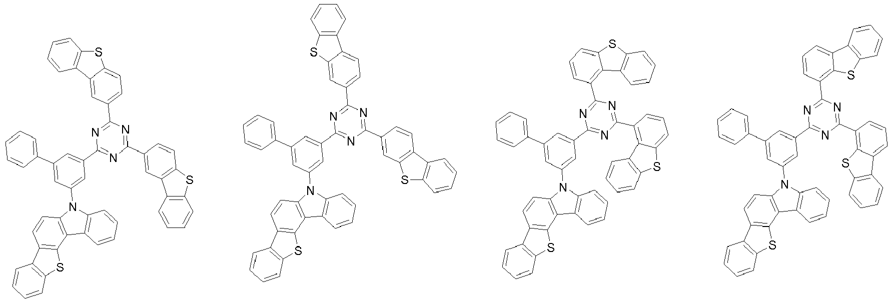

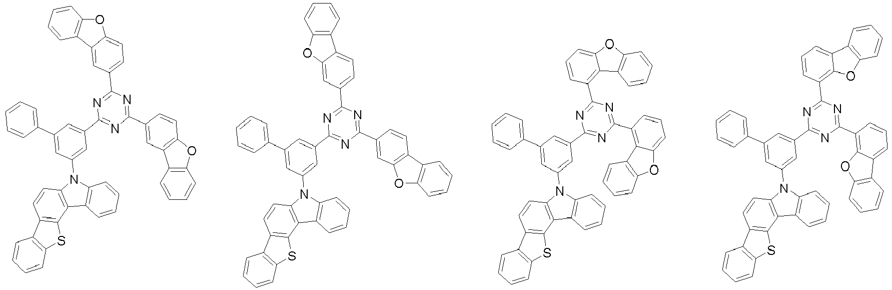

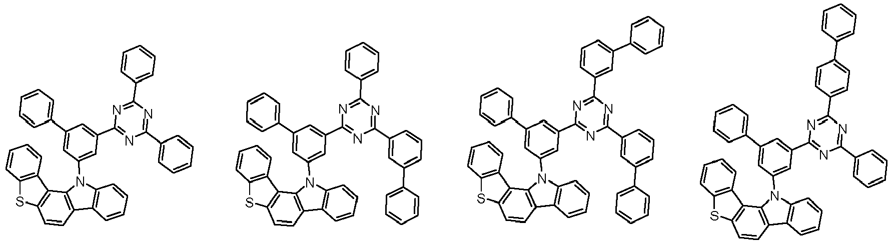

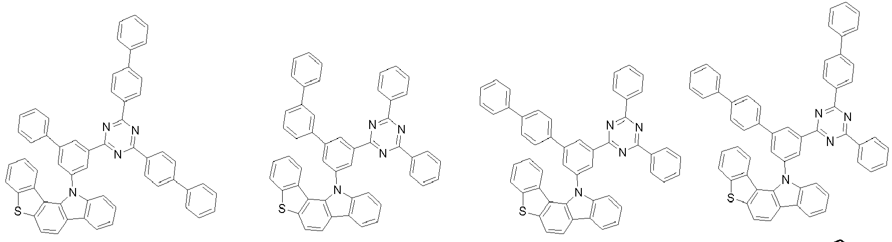

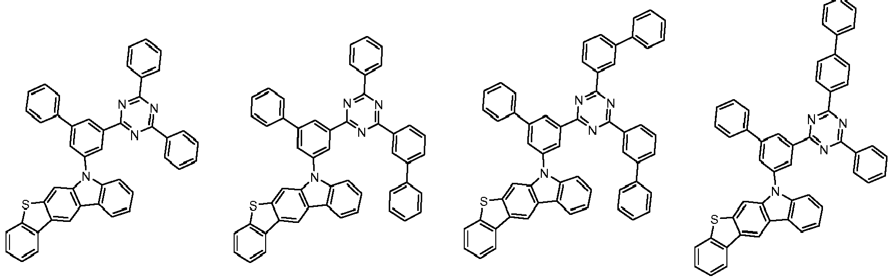

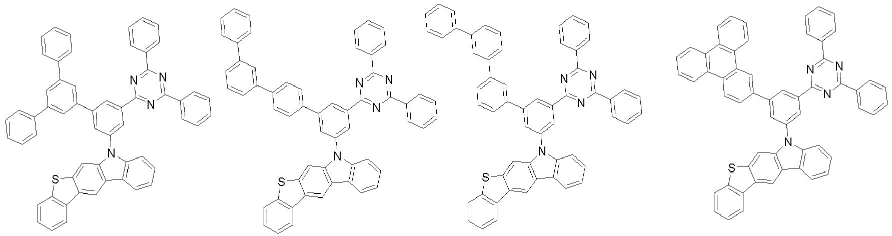

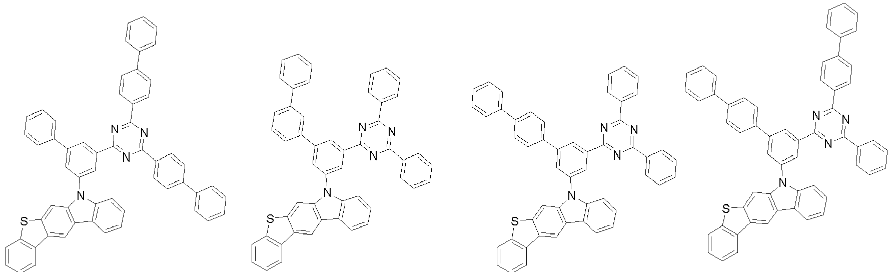

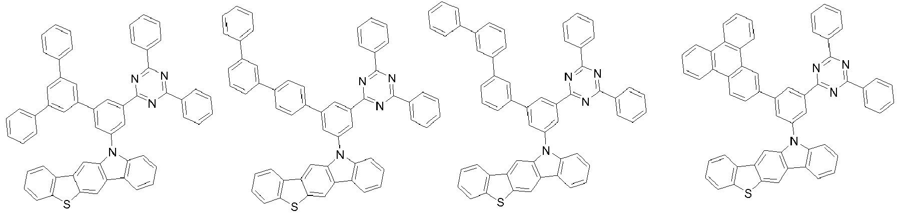

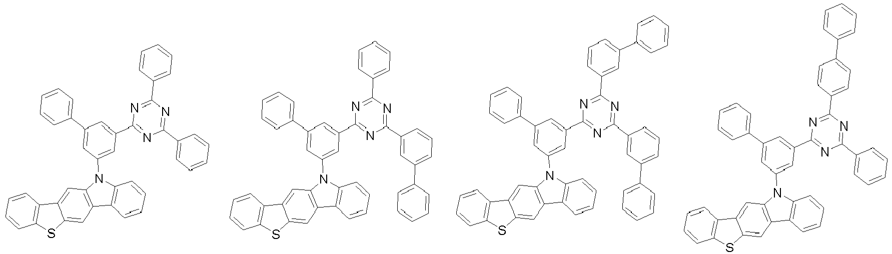

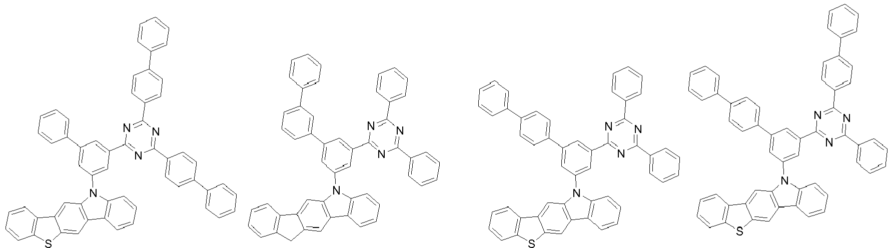

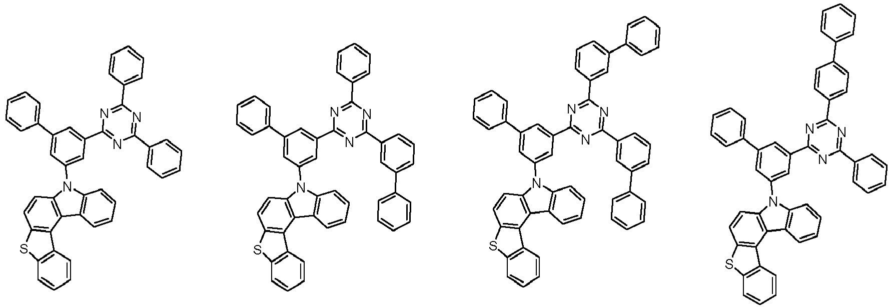

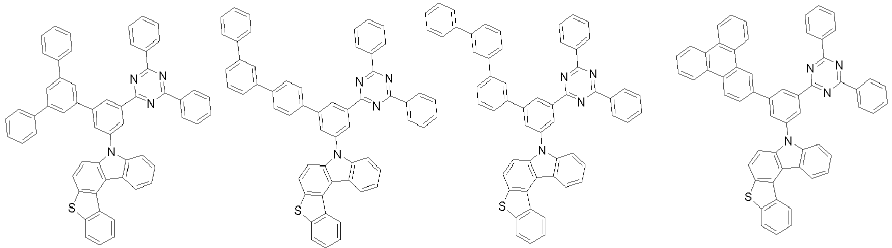

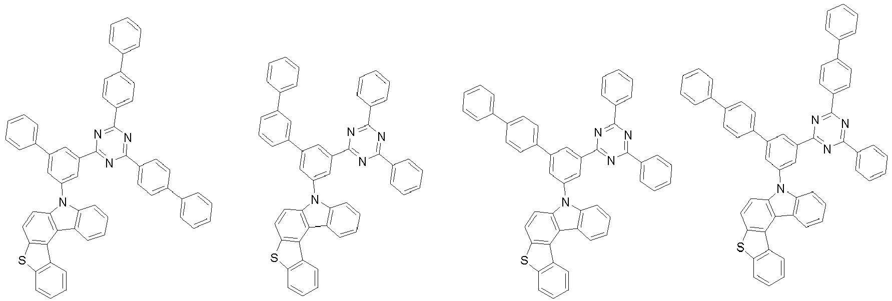

### Synthesis of the compounds of formulae (Ia) and (Ib)

The compounds of formulae (Ia) and (Ib) are for example prepared by one of the following processes:

### Process A:

A process for preparing a compound of formula (Ia) or (lb), comprising the steps:
(a) Coupling a compound of formula (Ia1) or (Ib1) 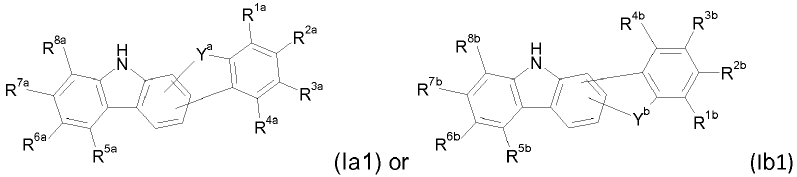 with a compound of formula (IIIa) or (IIIb) 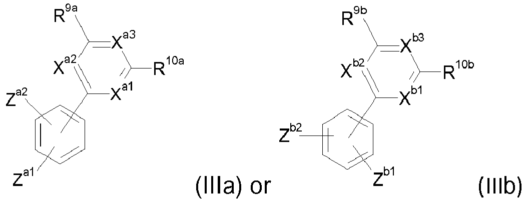 wherein
   Z^{a1}, Z^{a2}, Z^{b1} and Z^{b2} each independently represents a halide, preferably selected from the group consisting of I, F, Cl and Br, or a pseudohalide, preferably selected from the group consisting of mesylate, triflate, tosylate and nonaflate, -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring, or -MgX, wherein X is halide,
   whereby a compound of formula (Ia2) or (Ib2) is obtained 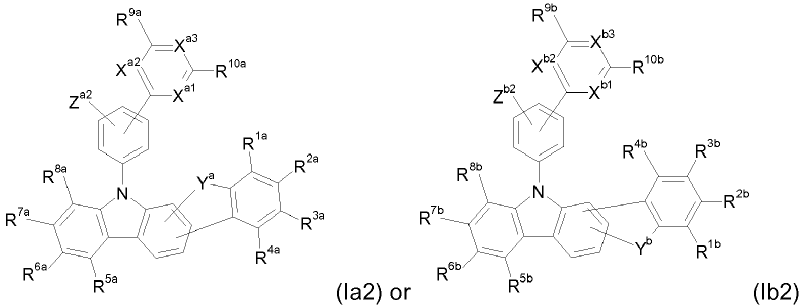
(b) Coupling a compound of formula (Ia2) or (Ib2)
   with a compound of formula (IVa) or (IVb)
   R^{11a}-Z^{a3} (IVa) or
   R^{11b}-Z^{b3} (IVb)
   wherein
   Z^{a3} and Z^{b3} each independently represents a halide, preferably selected from the group consisting of I, F, Cl and Br, or a pseudohalide, preferably selected from the group consisting of mesylate, triflate, tosylate and nonaflate, -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring, or -MgX, wherein X is halide,
   whereby a compound of formula (Ia) or (Ib) is obtained.

### Process B:

A process for preparing a compound of formula (Ia) or (Ib) comprising the steps:
(a) Coupling a compound of formula (IIIa) or (IIIb) 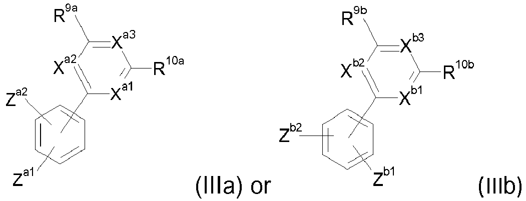 wherein
   Z^{a1}, Z^{a2}, Z^{b1} and Z^{b2} each independently represents a halide, preferably selected from the group consisting of I, F, Cl and Br, or a pseudohalide, preferably selected from the group consisting of mesylate, triflate, tosylate and nonaflate, -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring, or -MgX, wherein X is halide,
   with a compound of formula (IVa) or (IVb)
   R^{11a}-Z^{a3} (IVa) or
   R^{11b}-Z^{b3} (IVb)
   wherein
   Z^{a3} and Z^{b3} each independently represents a halide, preferably selected from the group consisting of I, F, Cl and Br, or a pseudohalide, preferably selected from the group consisting of mesylate, triflate, tosylate and nonaflate, -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring, or -MgX, wherein X is halide,
   whereby a compound of formula (Ia3) or (Ib3) is obtained 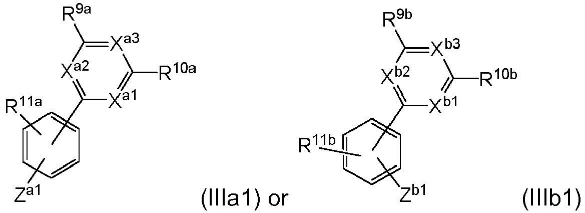
(b) Coupling a compound of formula (IIIa1) or (IIIb1)
   with a compound of formula (Ia1) or (Ib1) 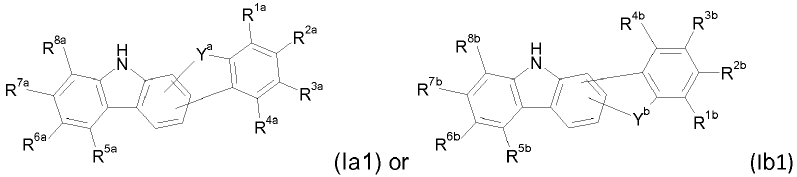 whereby a compound of formula (Ia) or (Ib) is obtained.

### Process C:

A process for preparing a compound of formula (Ia) or (Ib) comprising the steps:
(a) Coupling a compound of formula (Va) or (Vb) 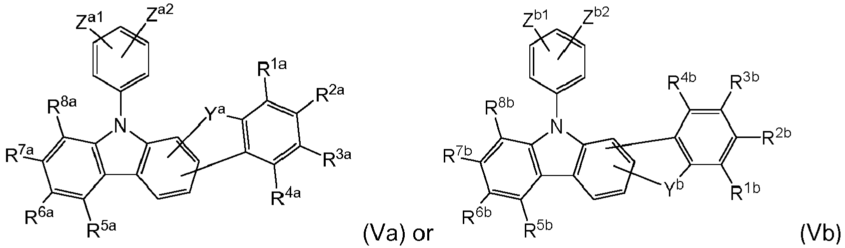 wherein
   Z^{a1}, Z^{a2}, Z^{b1} and Z^{b2} each independently represents a halide, preferably selected from the group consisting of I, F, Cl and Br, or a pseudohalide, preferably selected from the group consisting of mesylate, triflate, tosylate and nonaflate; -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring; or -MgX, wherein X is halide,
   with a compound of formula (VIa) or (VIb) 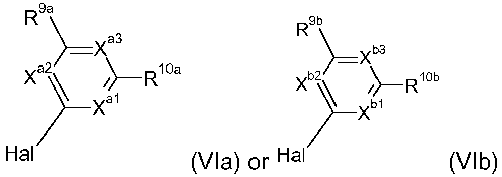
   wherein
   Hal is a halide, preferably selected from the group consisting of I, F, Cl and Br, or a pseudohalide, preferably selected from the group consisting of mesylate, triflate, tosylate and nonaflate,
   whereby a compound of formula (Ia2) or (Ib2) is obtained 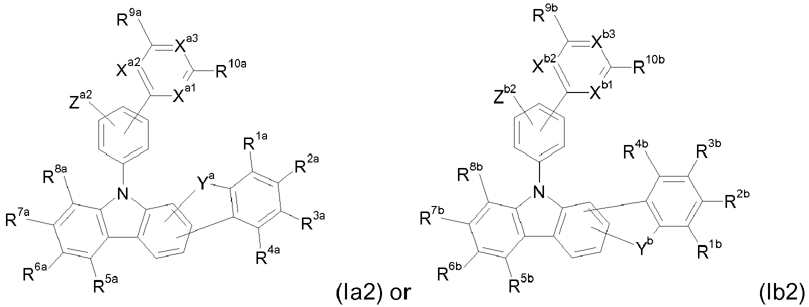
(b) Coupling a compound of formula (Ia2) or (Ib2)
   with a compound of formula (IVa) or (IVb)
   R^{11a}-Z^{a3} (IVa) or
   R^{11b}-Z^{b3} (IVb)
   wherein
   Z^{a3} and Z^{b3} each independently represents a halide, preferably selected from the group consisting of I, F, Cl and Br, or a pseudohalide, preferably selected from the group consisting of mesylate, triflate, tosylate and nonaflate; -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring; or -MgX, wherein X is halide,
   whereby a compound of formula (Ia) or (Ib) is obtained.

### Process D:

A process for preparing a compound of formula (Ia) or (Ib) comprising the steps:
(a) Coupling a compound of (Va) or (Vb) 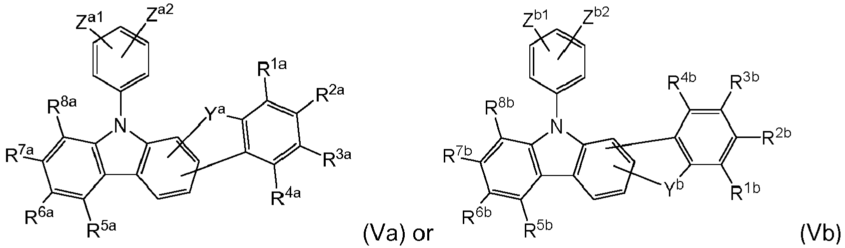 wherein
   Z^{a1}, Z^{a2}, Z^{b1} and Z^{b2} each independently represents a halide, preferably selected from the group consisting of I, F, Cl and Br, or a pseudohalide, preferably selected from the group consisting of mesylate, triflate, tosylate and nonaflate; -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring; or -MgX, wherein X is halide,
   with a compound of formula (IVa) or (IVb)
   R^{11a}-Z^{a3} (IVa) or
   R^{11b}-Z^{b3} (IVb)
   wherein
   Z^{a3} and Z^{b3} each independently represents a halide, preferably selected from the group consisting of I, F, Cl and Br, or a pseudohalide, preferably selected from the group consisting of mesylate, triflate, tosylate and nonaflate; -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring; or -MgX, wherein X is halide,
   whereby a compound of formula (VIIa) or (VIIb) is obtained. 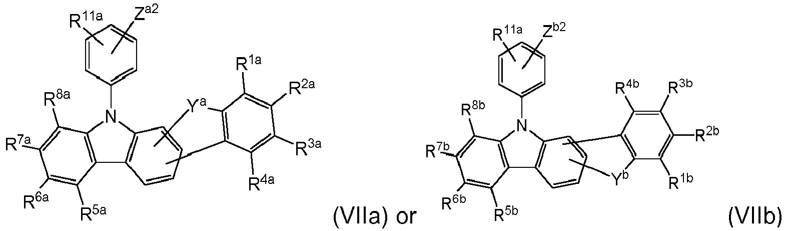
(b) Coupling a compound of formula (VIIa) or (VIIb)
   with a compound of formula (VIa) or (Vlb) 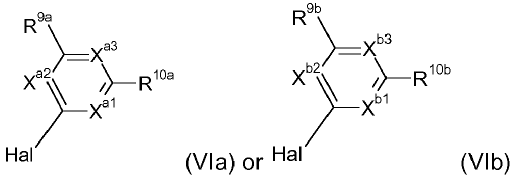 wherein
   Hal is a halide, preferably selected from the group consisting of I, F, Cl and Br, or a pseudohalide, preferably selected from the group consisting of mesylate, triflate, tosylate and nonaflate,
   whereby a compound of formula (Ia) or (Ib) is obtained.

The groups and residues and preferred groups and residues R^{1a}, R^{2a}, R^{3a}, R^{4a}, R^{5a}, R^{6a}, R^{7a}, R^{8a}, R^{9a}, R^{10a}, R^{11a}, X^{a1}, X^{a2}, X^{a3} and Y^{a}, R^{1b}, R^{2b}, R^{3b}, R^{4b}, R^{5b}, R^{6b}, R^{7b}, R^{8b}, R^{9b}, R^{10b}, R^{11b}, X^{b1}, X^{b2}, X^{b3} and Y^{b} are defined above.

Details of the reaction steps and process conditions are mentioned in the examples of the present application. The production method of the compounds of formula (Ia) and (Ib) according to the present invention is not particularly limited and it is produced according to known methods, for example, by a Suzuki coupling as described in Journal of American Chemistry Society 121 (1999) 9550 to 9561 or Chemical Reviews 95 (1995) 2457 to 2483 or Kumada coupling described in Org. Lett., 2010, 12, 2298-2301 or Angew. Chem., 2002, 114, 4218-4221. Coupling reactions involving a N atom and a C atom, so called amination reactions can be carried out in the presence of copper in an Ullmann type coupling such as described in Org. Lett., 2002, 4, 581-584 or Org. Lett., 2002, 4, 581-584 or a Chan Lam type coupling described in Org. Lett., 2003, 5, 4397-4400 or Org. Lett., 2013, 15, 1544-1547. Amination reactions can also be carried out in the presence of palladium in so-called Buchwald-Hartwig coupling such as described in Org. Lett., 2008, 10, 4109-4112, and Org. Lett., 2003, 5, 2413-2415. Amination reactions can also be carried out between amines and aryl fluorides be nucleophilic aromatic substitution such as described in Synthesis, 48(5), 737-750; 2016 or Angewandte Chemie, International Edition, 51(32), 8012-8016.

### Combination of at least one compound of formula (Ia) or (Ib) and at least one compound of formula (II) in organic electronics applications

It has been found that the combination of at least one compound of formula (Ia) or (Ib) and at least one compound of formula (II) (especially a composition comprising at least one compound of formula (Ia) or (Ib) and at least one compound of formula (II)) is particularly suitable for use in applications in which charge carrier conductivity is required.

A further subject of the present invention is therefore directed to a composition comprising
i) at least one polycyclic compound represented by formula (Ia) or (Ib) according to the present invention, and
ii) at least one compound of formula (II) 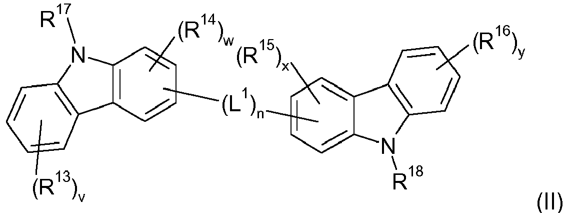 wherein
   R¹³, R¹⁴, R¹⁵ and R¹⁶ each independently represents hydrogen, a halogen atom, a substituted or unsubstituted alkyl group having 1 to 25 carbon atoms, a substituted or unsubstituted alkenyl group having 2 to 25 carbon atoms, a substituted or unsubstituted alkynyl group having 2 to 25 carbon atoms, a substituted or unsubstituted cycloalkyl group having 3 to 25 carbon atoms, a substituted or unsubstituted alkoxy group having 1 to 25 carbon atoms, a substituted or unsubstituted aryl group having 6 to 24 carbon atoms, a substituted or unsubstituted heterocyclic group having 5 to 30 ring atoms, a substituted or unsubstituted aryloxy group having 6 to 24 carbon atoms, a substituted or unsubstituted alkylthio group having 1 to 25 carbon atoms, a substituted or unsubstituted arylthio group having 6 to 24 carbon atoms, alkyl and/or aryl substituted silyl group, alkyl and/or aryl substituted carbonyl group, a substituted phosphoryl group, or CN;
   v and y are each independently 0, 1, 2, 3 or 4;
   w and x are each independently 0, 1, 2 or 3;
   R¹⁷ and R¹⁸ are independently a substituted or unsubstituted aromatic hydrocarbon group having 6 to 30 ring carbon atoms or a substituted or unsubstituted heterocyclic group having 5 to 30 ring atoms, an alkyl group having 1 to 25 carbon atoms, a substituted or unsubstituted cycloalkyl group having a ring formed of 3 to 25 carbon atoms, a substituted or unsubstituted aralkyl group having 7 to 24 carbon atoms;
   L¹ is a direct bond, a substituted or unsubstituted alkylene group having 1 to 30 carbon atoms, a substituted or unsubstituted cycloalkylene group having a ring structure formed of 3 to 20 carbon atoms, a divalent silyl group having 2 to 20 carbon atoms, a substituted or unsubstituted divalent aromatic hydrocarbon group having a ring structure formed of 6 to 30 carbon atoms, or a substituted or unsubstituted divalent heterocyclic group having a ring structure formed of 5 to 30 atoms;
   n is 1, 2 or 3; in the case that n is 2 or 3, L₁ is the same or different in each occurrence.

The content ratio of the at least one compound of formula (Ia) or (Ib) and the at least one compound of formula (II) in the composition comprising at least one compound of formula (Ia) or (Ib) and at least one compound of formula (II) is not particularly limited. The ratio of the at least one compound of formula (Ia) or (Ib) : the at least one compound of formula (II) is preferably 1:99 to 99:1, more preferably 10:90 to 90:10, most preferably 30:70 to 70:30, each based on the mass of the composition.

A further subject of the present invention is directed to an organic electronic device, comprising a composition comprising at least one compound of formula (Ia) or (Ib) and at least one compound of formula (II) according to the present invention.

The composition comprising at least one compound of formula (Ia) or (Ib) and at least one compound of formula (II) can in principle be used in any layer of an EL device, but is preferably used as host, charge transport, especially electron transport, and/or charge blocking, especially hole blocking, material. Particularly, the composition comprising at least one compound of formula (Ia) or (Ib) and at least one compound of formula (II) is used as host material, hole blocking material and/or electron transport material for phosphorescence or fluorescence emitters. More preferably, the composition comprising at least one compound of formula (Ia) or (Ib) and at least one compound of formula (II) is used as host material for phosphorescence or fluorescence emitters. Most preferably, the composition comprising at least one compound of formula (Ia) or (Ib) and at least one compound of formula (II) is used as host material for phosphorescence emitters.

Hence, a further subject of the present invention is directed to a material for an organic electroluminescence device comprising composition comprising at least one compound of formula (Ia) or (Ib) and at least one compound of formula (II) according to the present invention.

A further subject of the present invention is directed to a composition film comprising a composition comprising at least one compound of formula (Ia) or (Ib) and at least one compound of formula (II) according to the present invention.

A further subject of the present invention is directed to an organic electroluminescence device which comprises an organic thin film layer between a cathode and an anode, wherein the organic thin film layer comprises one or more layers and comprises a light emitting layer, and a composition comprising at least one compound of formula (Ia) or (Ib) and at least one compound of formula (II) according to the present invention.

A further subject of the present invention is directed to an emitting layer, a composition comprising at least one compound of formula (Ia) or (Ib) and at least one compound of formula (II) according to the present invention. In said embodiment the compound of formula (Ia) or (Ib) is preferably used as host material or as co-host material together with one or more, preferably one, compound of formula (II) as further host material. More preferably, a composition comprising at least one compound of formula (Ia) or (Ib) as host material and at least one compound of formula (II) as co-host material together with a phosphorescent emitter is used.

A further subject of the present invention is directed to the use of a composition comprising at least one compound of formula (Ia) or (Ib) and at least one compound of formula (II) according to the present invention in an organic electroluminescence device.

A further subject of the present invention is directed to an electronic equipment comprising the organic electroluminescence device according the present invention.

### R¹³, R¹⁴, R¹⁵ and R¹⁶

Preferably, R¹³, R¹⁴, R¹⁵ and R¹⁶ each independently represent hydrogen, a substituted or unsubstituted alkyl group having 1 to 25 carbon atoms, a substituted or unsubstituted cycloalkyl group having 3 to 25 carbon atoms, or a substituted or unsubstituted aryl group having 6 to 24 carbon atoms, or a substituted or unsubstituted heterocyclic group having 5 to 30 ring atoms, F or CN.

More preferably, R¹³, R¹⁴, R¹⁵ and R¹⁶ each independently represent hydrogen, a methyl group, an ethyl group, a n-propyl group, an isopropyl group, a n-butyl group, a s-butyl group, an isobutyl group, a t-butyl group, a cyclohexyl group, F, CN, or a phenyl group, wherein the groups mentioned before are unsubstituted or substituted, preferably unsubstituted.

Suitable substituents are mentioned above.

Most preferably, R¹³, R¹⁴, R¹⁵ and R¹⁶ are hydrogen.

v and y are each independently preferably, 0, 1 or 2, more preferably 0 or 1 and most preferably 0.

w and x are each independently preferably, 0, 1 or 2, more preferably 0 or 1 and most preferably 0.

In a further preferred embodiment, v, y, w and x are 0, or one of v, y, w and x is 1 and the remaining of v, y, w and x are 0. Most preferably, v, y, w and x are 0.

### The group -(L¹)ₙ-

Preferably, L¹ represents a direct bond, a substituted or unsubstituted divalent aromatic hydrocarbon group having a ring structure formed of 6 to 30 carbon atoms, or a substituted or unsubstituted divalent heterocyclic group having a ring structure formed of 5 to 30 atoms.

More preferably, L¹ represents a direct bond, divalent substituted or unsubstituted phenyl, divalent substituted or unsubstituted biphenyl, divalent substituted or unsubstituted naphthyl, divalent substituted or unsubstituted carbazolyl, divalent substituted or unsubstituted dibenzothiophenyl or divalent substituted or unsubstituted dibenzofuranyl.

Most preferably, -(L¹)ₙ- is a direct bond.

### R¹⁷, R¹⁸

Preferably, R¹⁷ and R¹⁸ are independently a substituted or unsubstituted aromatic hydrocarbon group having 6 to 30 ring carbon atoms or a substituted or unsubstituted heterocyclic group having 5 to 30 ring atoms.

More preferably, R¹⁷ and R¹⁸ are independently a phenyl group, a naphthyl group, a phenanthryl group, a biphenyl group, a terphenyl group, a quaterphenyl group, a triphenylenyl group, a fluorenyl group, a spirofluorenyl group, a 9,9-diphenylfluorenyl group, a 9,9'-spirobi[9H-fluorene]-2-yl group, a 9,9-dimethylfluorenyl group, a dibenzofuranyl group, a N-phenylcarbazolyl group, or a dibenzothiophenyl group, wherein the groups mentioned before are unsubstituted or substituted. Suitable substituents are mentioned above.

Below, examples for compounds of formula (II) are given:

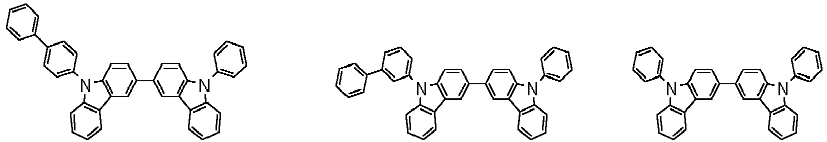

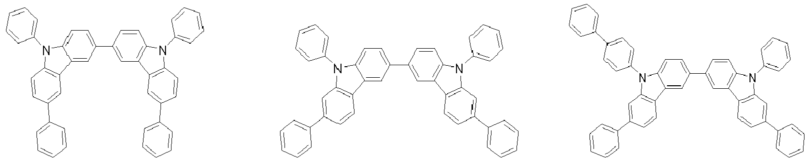

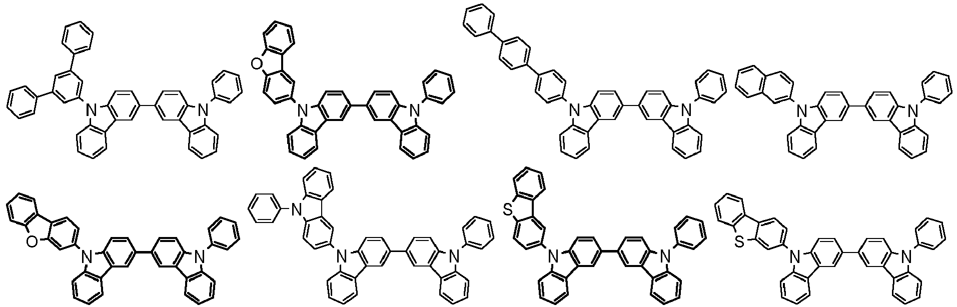

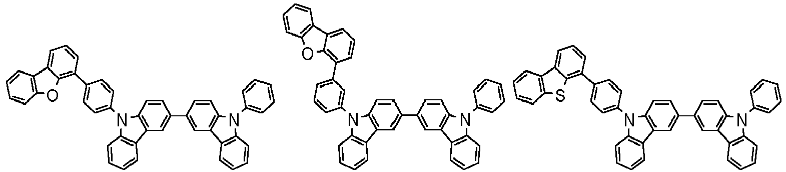

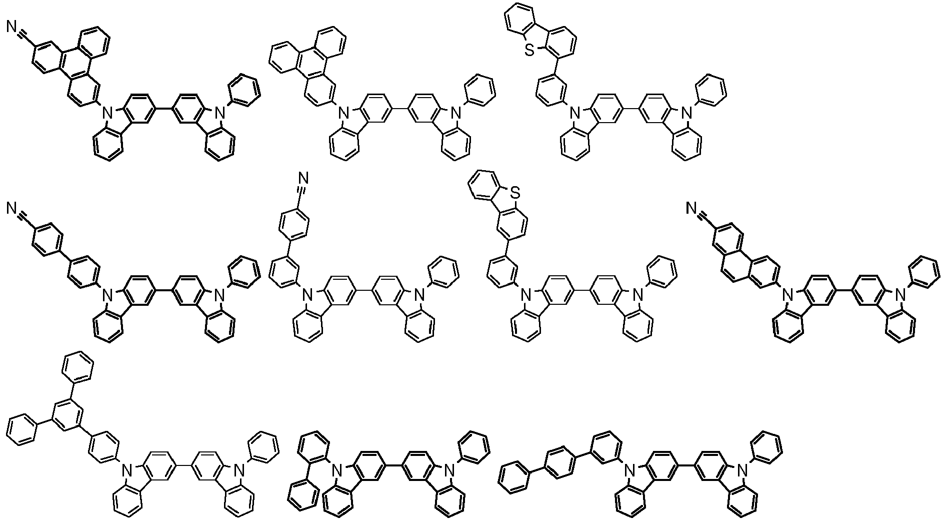

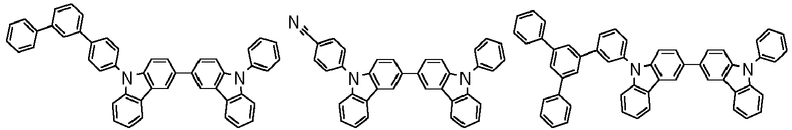

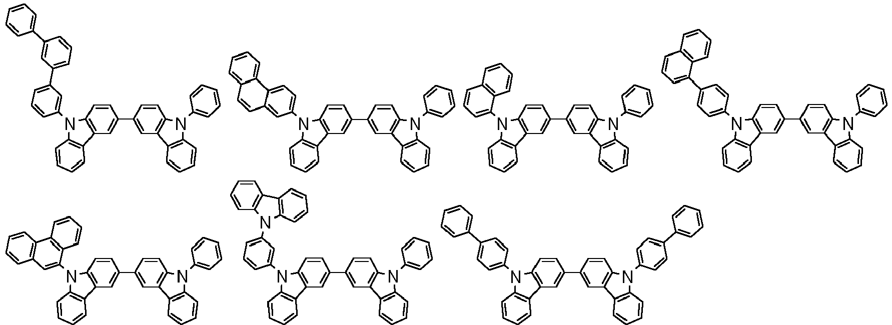

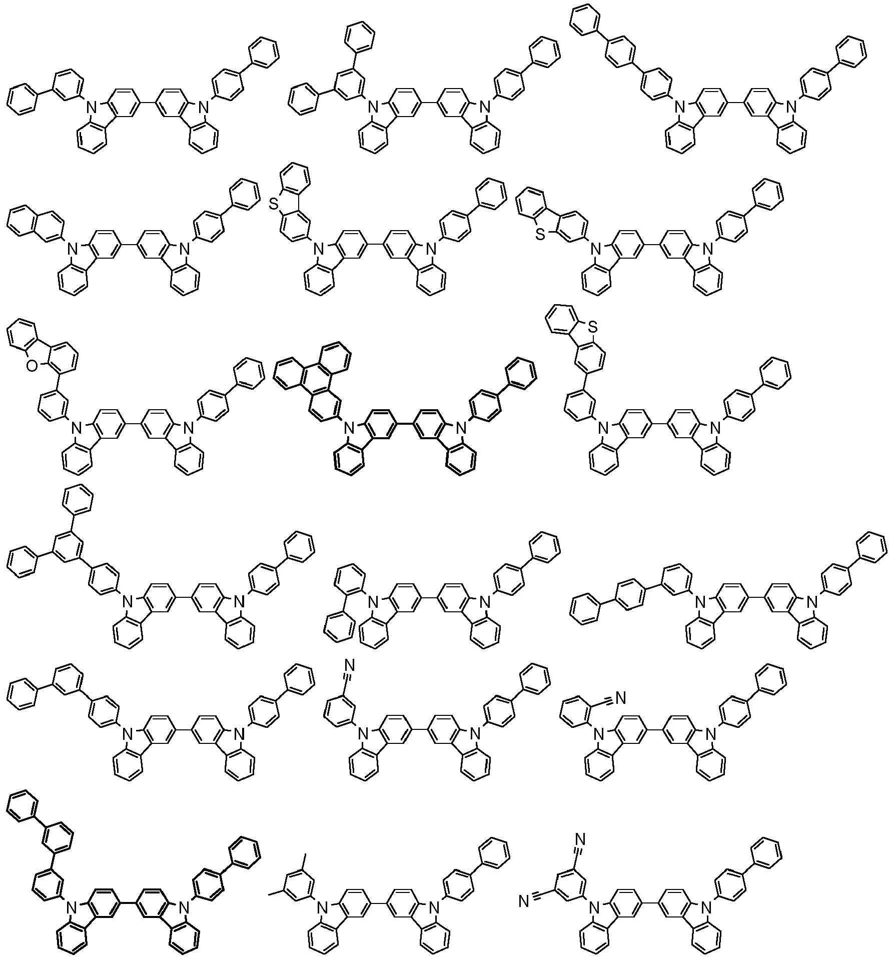

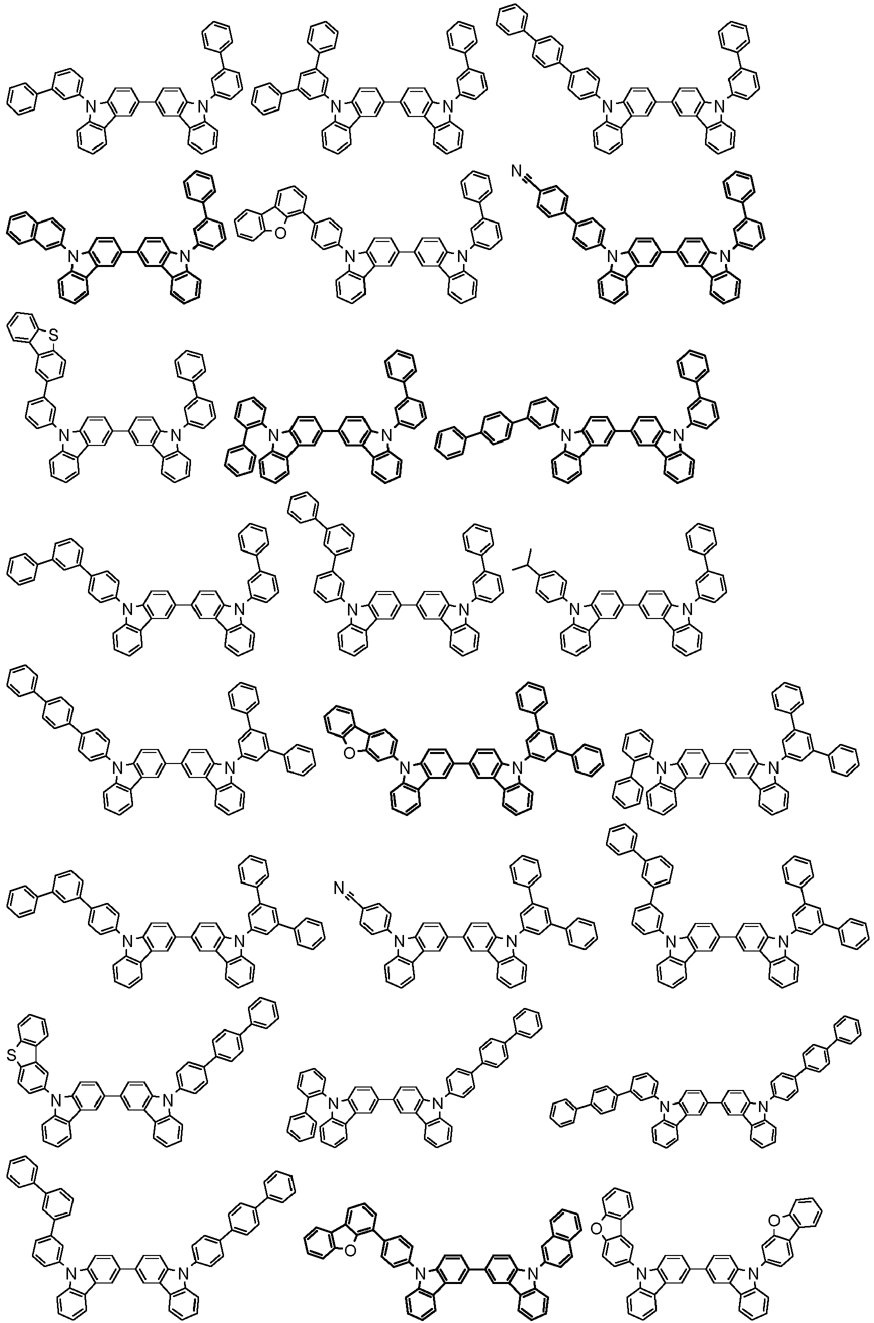

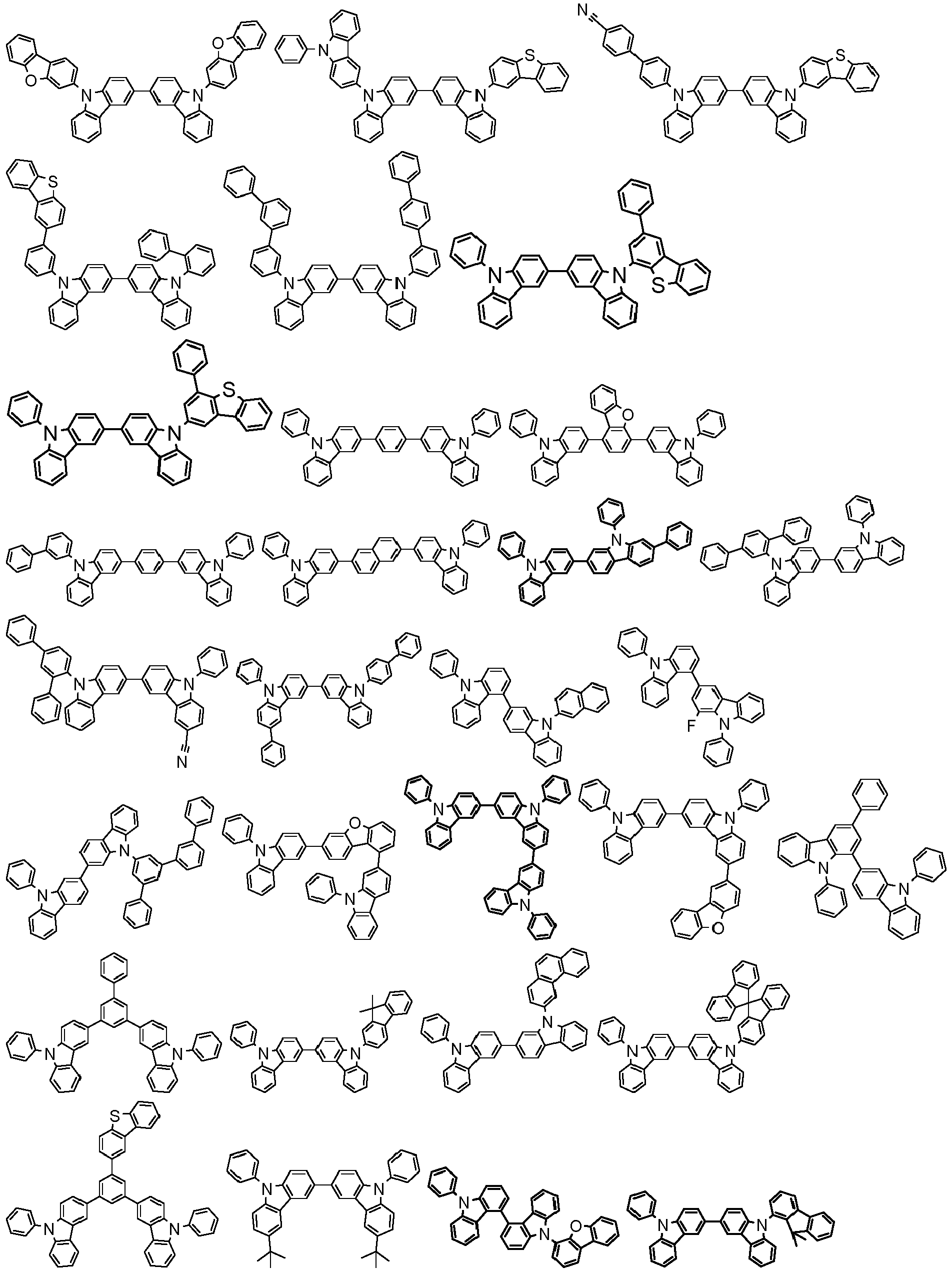

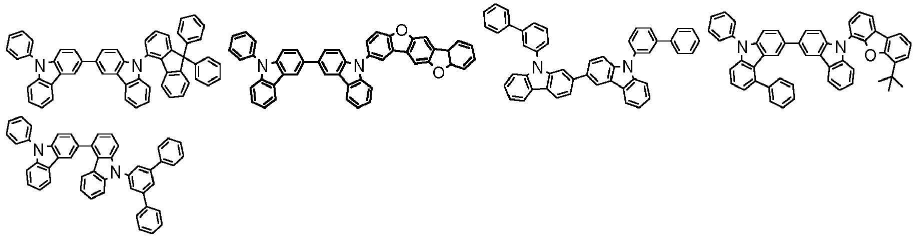

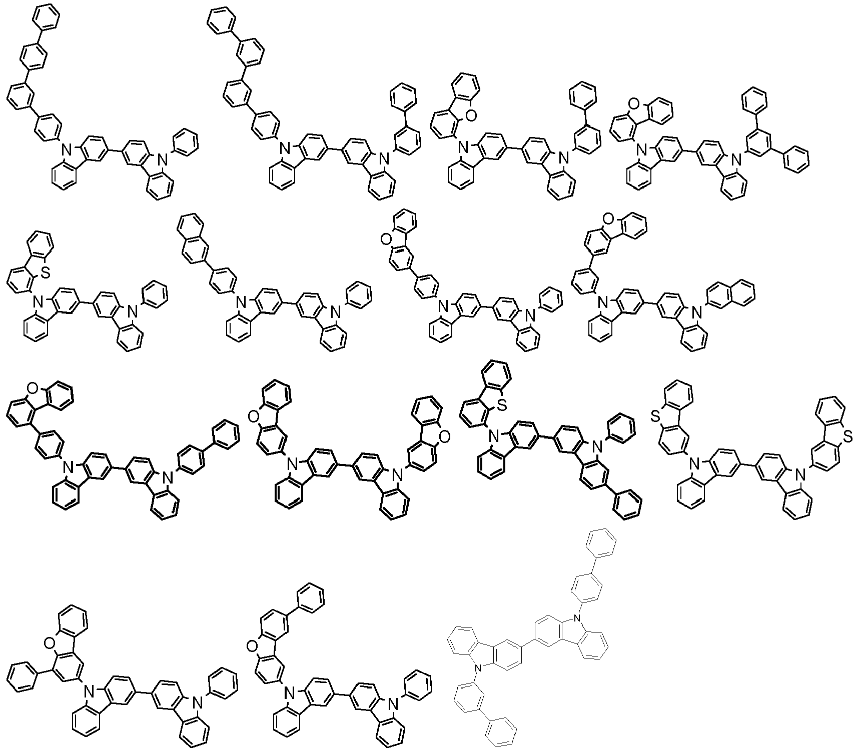

### Synthesis of the compounds of formula (II)

The preparation of the compounds of formula (II) is known in the art, and for example described in WO2013/062075 and WO2013/084881.

### Compounds of formula (Ia) or (Ib) and compositions comprising at least one compound of formula (Ia) or (Ib) and at least one compound of formula (II) in organic electronics applications

It has been found that the compounds of formulae (Ia) and (Ib) and the compositions comprising at least one compound of formula (Ia) or (Ib) and at least one compound of formula (II) are particularly suitable for use in applications in which charge carrier conductivity is required, especially for use in organic electronics applications, for example selected from switching elements such as organic transistors, e.g. organic FETs and organic TFTs, organic solar cells and organic light-emitting diodes (OLEDs).

The term organic EL device (organic electroluminescence device) is used interchangeably with the term organic light-emitting diode (OLED) in the following; i.e. both terms have the same meaning in the sense of the present application.

The present invention further relates to a material for an organic EL device comprising at least one compound of formula (Ia) or (lb), or a composition comprising at least one compound of formula (Ia) or (Ib) and at least one compound of formula (II).

The organic transistor generally includes a semiconductor layer formed from an organic layer with charge transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor. The layers with charge transport capacity may comprise the compound of formula (Ia) or (lb), or the composition comprising at least one compound of formula (Ia) or (Ib) and at least one compound of formula (II).

The organic solar cell (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or charge transport capacity. In addition, it is possible for further layers known to those skilled in the art to be present in the organic solar cell. The layers with charge transport capacity may comprise the compound of formula (Ia) or (lb), or the composition comprising at least one compound of formula (Ia) or (Ib) and at least one compound of formula (II).

The compounds of formulae (Ia) and (lb), or the composition comprising at least one compound of formula (Ia) or (Ib) and at least one compound of formula (II) being particularly suitable in OLEDs for use as matrix material (host material) in a light-emitting layer and/or as charge and/or exciton blocker material, i.e. as electron/exciton blocker material or as hole/exciton blocker material, and/or charge transport material, i.e. hole transport material or electron transport material, preferably as matrix material in a light-emitting layer and/or as electron transport material, especially in combination with a phosphorescence emitter.

In the case of use of the inventive compounds of formulae (Ia) and (lb), or the composition comprising at least one compound of formula (Ia) or (Ib) and at least one compound of formula (II) in OLEDs, OLEDs having good overall properties, preferably a long lifetime and/or a low driving voltage are obtained. The inventive compounds of formulae (Ia) and (lb), or the composition comprising at least one compound of formula (Ia) or (Ib) and at least one compound of formula (II) are suitable especially for use as matrix and/or charge transport, i.e. hole or electron transport, and/or charge blocker material, i.e. hole or electron blocker material. Furthermore, the compounds of formulae (Ia) and (lb), or the composition comprising at least one compound of formula (Ia) or (Ib) and at least one compound of formula (II) can be used as conductor/complementary materials in organic electronic applications selected from switching elements and organic solar cells. (In the sense of the present application, the terms matrix and host are used interchangeable).

### Organic EL device (OLED)

The organic EL device as one embodiment of the invention comprises one or more organic thin film layers including an emitting layer between a cathode and an anode, and at least one layer of the organic thin film layers comprises the compound of formula (Ia) or (lb), or the composition comprising at least one compound of formula (Ia) or (Ib) and at least one compound of formula (II).

As examples of the organic thin film layers that comprise the compound of formula (Ia) or (lb), or the composition comprising at least one compound of formula (Ia) or (Ib) and at least one compound of formula (II), an anode-side organic thin film layer (hole-transporting layer, hole-injecting layer, or the like), an emitting layer, a cathode-side organic thin film layer (electron-transporting layer, electron-injecting layer, or the like) provided between a cathode and an emitting layer, a spacing layer, a barrier layer or the like can be given. The examples are not limited thereto.

The compound of formula (Ia) or (lb), or the composition comprising at least one compound of formula (Ia) or (Ib) and at least one compound of formula (II) may be contained in any of the abovementioned layers, and can be used as a host material or a dopant material in the emitting layer of a fluorescent emitting unit, a host material in the emitting layer of a phosphorescent emitting unit, a hole-transporting layer, an electron-transporting layer or the like of an emitting unit.

Preferably, the light emitting layer comprises at least one compound of formula (Ia) or (lb), or the composition comprising at least one compound of formula (Ia) or (Ib) and at least one compound of formula (II) according to the present invention.

Preferably, the compounds of formula (Ia) or (lb), or the compositions comprising at least one compound of formula (Ia) or (Ib) and at least one compound of formula (II) are used as matrix materials (host materials), preferably in an emitting layer of an OLED, more preferably in an emitting layer of an OLED comprising at least one compound of formula (Ia) or (lb), or the composition comprising at least one compound of formula (Ia) or (Ib) and at least one compound of formula (II) and at least one emitter material, wherein the emitter material is preferably a fluorescent or phosphorescent emitter material, more preferably a blue, green or red fluorescent or phosphorescent emitter material, most preferably a green or red phosphorescent emitter material, further most preferably a green phosphorescent emitter material.

According to another embodiment, the compounds of formula (Ia) or (lb), or the composition comprising at least one compound of formula (Ia) or (Ib) and at least one compound of formula (II) are preferably used in the electron transporting layer of an OLED.

The present invention therefore further relates to the organic electroluminescence device according to the present invention, wherein an electron transporting layer is provided between the cathode and the light emitting layer, and the electron transporting layer comprises at least one compound of formula (Ia) or (lb), or the composition comprising at least one compound of formula (Ia) or (Ib) and at least one compound of formula (II) according to the present invention.

According to another embodiment, the compounds of formula (Ia) or (lb), or the composition comprising at least one compound of formula (Ia) or (Ib) and at least one compound of formula (II) are preferably used in the hole blocking layer of an OLED.

The present invention therefore further relates to the organic electroluminescence device according to the present invention, wherein a hole blocking layer is provided between the electron transporting layer and the light emitting layer, and the hole blocking layer comprises at least one compound of formula (Ia) or (lb), or the composition comprising at least one compound of formula (Ia) or (Ib) and at least one compound of formula (II) according to the present invention.

The organic EL device of the invention may be a fluorescent or phosphorescent monochromatic emitting device or may be a fluorescent/phosphorescent hybrid white emitting device. It may be a simple emitting device having a single emitting unit or a tandem emitting device having plural emitting units. Among them, the organic EL device may preferably be a phosphorescent emitting device.
As the representative device structure of a simple type organic EL device, the following device configuration can be given.

### (1) Anode/emitting unit/cathode

The emitting unit mentioned above may be a stacked type emitting unit comprising plural phosphorescent emitting layers or plural fluorescent emitting layers. In this case, in order to prevent diffusion of excitons generated in the phosphorescent emitting layer to the fluorescent emitting layer, a spacing layer may be provided between the emitting layers. The representative layer configuration of the emitting unit is given below.
(a) Hole-transporting layer/Emitting layer (/Electron-transporting layer)
(b) Hole-transporting layer/First phosphorescent emitting layer/Second phosphorescent emitting layer (/Electron-transporting layer)
(c) Hole-transporting layer/Phosphorescent emitting layer/Spacing layer/Fluorescent emitting layer (/Electron-transporting layer)
(d) Hole-transporting layer/First phosphorescent emitting layer/Second phosphorescent emitting layer/Spacing layer/Fluorescent emitting layer (/Electron-transporting layer)
(e) Hole-transporting layer/First phosphorescent emitting layer/Spacing layer/Second phosphorescent emitting layer/Spacing layer/Fluorescent emitting layer (/Electron-transporting layer)
(f) Hole-transporting layer/Phosphorescent emitting layer/Spacing layer/First fluorescent emitting layer/Second fluorescent emitting layer (/Electron-transporting layer)
(g) Hole-transporting layer/Electron barrier layer/Emitting layer (/Electron-transporting layer)
(h) Hole-transporting layer/Emitting layer/Hole barrier layer (/Electron-transporting layer)
(i) Hole-transporting layer/Fluorescent emitting layer/Triplet barrier layer (/Electron-transporting layer)

The phosphorescent or fluorescent emitting layer as mentioned above can emit different colors of light. Specifically, in the stacked emitting layer (d), a layer configuration of the hole transporting layer/first phosphorescent emitting layer (red emission)/second phosphorescent emitting layer (green emission)/spacing layer/fluorescent emitting layer (blue emission)/electron transporting layer or the like can be given.

Between each emitting layer and the hole-transporting layer or the spacing layer, an electron barrier layer may be provided appropriately. Between each emitting layer and the electron transporting layer, a hole-barrier layer (a hole blocking layer) may be provided appropriately. Due to provision of an electron-barrier layer or a hole-barrier layer, electrons or holes can be confined within the emitting layer, whereby possibility of recombination of carriers in the emitting layer can be increased, and the life can be improved.

As the represented device configuration of a tandem organic EL device, the following device configuration can be given.

### (2) Anode/first emitting unit/intermediate layer/second emitting unit/cathode

Here, as the first emitting unit and the second emitting unit, the same emitting units as those mentioned above can independently be given, for example.

In general, the intermediate layer is called an intermediate electrode, an intermediate conductive layer, a carrier-generating layer, an electron-withdrawing layer, and a known material configuration that supplies electrons to the first emitting unit and supplies holes to the second emitting unit can be used.

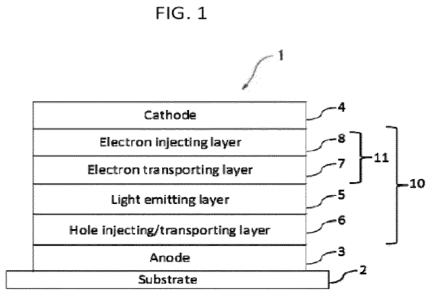

FIG. 1 shows a schematic configuration of one example of the organic EL device of the invention. The organic EL device 1 comprises a substrate 2, an anode 3, a cathode 4 and an emitting unit 10 provided between the anode 3 and the cathode 4. The emitting unit 10 comprises an emitting layer 5 preferably comprising a host material and a dopant. A hole injecting and transporting layer 6 or the like may be provided between the emitting layer 5 and the anode 3 and an electron-injecting layer 8 and an electron transporting layer 7 or the like (electron injecting and transporting unit 11) may be provided between the emitting layer 5 and the cathode 4. An electron-barrier layer may be provided on the anode 3 side of the emitting layer 5 and a hole-barrier layer may be provided on the cathode 4 side of the emitting layer 5. Due to such configuration, electrons or holes can be confined in the emitting layer 5, whereby possibility of generation of excitons in the emitting layer 5 can be improved.

Due to such configuration, electrons or holes can be confined in the emitting layer 5, whereby possibility of generation of excitons in the emitting layer 5 can be improved.

Herein, a host that is combined with a fluorescent dopant is referred to as a fluorescent host and a host that is combined with a phosphorescent dopant is referred to as a phosphorescent host. The fluorescent host and the phosphorescent host are not distinguished only by the molecular structure thereof. That is, the phosphorescent host means a material constituting a phosphorescent emitting layer that contains a phosphorescent dopant and does not mean a material that cannot be used as a material constituting a fluorescent dopant. The same can be applied to a fluorescent host.

### Substrate

The organic EL device is usually formed on a transparent substrate. The transparent substrate is a substrate for supporting the organic EL device, and is preferably a flat and smooth substrate having a 400-to-700-nm-visible-light transmittance of 50% or more. Specific examples thereof include glass plates and polymer plates. Examples of the glass plate include those obtained by using as raw materials soda-lime glass, barium/strontium-containing glass, lead glass, aluminosilicate glass, borosilicate glass, barium borosilicate glass, quartz, or the like. Examples of the polymer plate include those obtained by using as raw materials polycarbonate, acrylic polymer, polyethylene terephthalate, polyethersulfide, polysulfone, or the like.

### Anode

The anode of the organic EL device plays a role for injecting holes into its hole-transporting layer or emitting layer. It is effective to use one having a work function of 4.5 eV or more. As specific examples of the anode material, indium tin oxide alloy (ITO), tin oxide (NESA), indium zinc oxide, gold, silver, platinum, copper, and the like can be given. The anode can be formed by forming these electrode materials into a thin film by vapor deposition, sputtering or the like. In the case where emission from the emitting layer is taken out through the anode, the transmittance of the anode to the emission is preferably more than 10%. The sheet resistance of the anode is preferably several hundred Ω/□ or less. The film thickness of the anode, which varies depending upon the material thereof, is usually from 10 nm to 1 µm, preferably from 10 to 200 nm.

### Cathode

The cathode plays a role for injecting electrons into its electron-injecting layer, electron-transporting layer or emitting layer. The cathode is preferably formed of a material having a small work function. The cathode material is not particularly restricted. As specific examples of the cathode material, indium, aluminum, magnesium, a magnesium-indium alloy, a magnesium-aluminum alloy, an aluminum-lithium alloy, an aluminum-scandium-lithium alloy, a magnesium-silver alloy or the like can be given. As in the case of the anode, the cathode can be formed by forming the materials into a thin film by a deposition method, a sputtering method or the like. If necessary, emission can be outcoupled from the cathode side.

### Emitting layer

The present invention relates - in one embodiment - to an organic electroluminescence device, wherein the light emitting layer comprises at least one compound of formula (Ia) or (lb), or the composition comprising at least one compound of formula (Ia) or (Ib) and at least one compound of formula (II).

The emitting layer is an organic layer having an emitting function, and where a doping system is used, it usually comprises a host material and a dopant material (emitter).

The host material has a function of accelerating recombination of electrons and holes and confining excitons within the emitting layer. The dopant material has a function of emitting efficiently excitons obtained by recombination.

In the case of a phosphorescent device, the host material has a function of confining excitons mainly generated by a dopant within the emitting layer. The host material is preferably a compound of formula (Ia) or (lb), or a composition comprising at least one compound of formula (Ia) or (Ib) and at least one compound of formula (II) according to the invention.

In one preferred embodiment, in the emitting layer, a double host (also referred to as a host/cohost) that adjusts the carrier balance in the emitting layer may be used by combining an electron-transporting host and a hole-transporting host or by other methods. In said embodiment, the emitting layer comprises a first host material and a second host material and at least one component of the first host material and the second host material is a compound of the formula (Ia) or (Ib) according to the invention. Preferably, the emitting layer comprises a composition comprising at least one compound of formula (Ia) or (Ib) and at least one compound of formula (II) according to the invention.

Double dopant may be used in which two or more types of dopant materials having a high quantum yield are incorporated, and each dopant emits light. Specifically, by allowing a host, a red dopant and a green dopant to be co-deposited, yellow emission from the common emitting layer, whereby yellow emission is realized.

As for the emitting layer, by allowing plural emitting layers to be a stacked body, electrons and holes are accumulated in the interface of the emitting layers, whereby the recombination region is concentrated in the interface of the emitting layers. As a result, the quantum efficiency is improved.

Easiness in injection of holes to the emitting layer and easiness in injection of electrons to the emitting layer may differ. Further, the hole-transporting performance and the electron transporting performance indicated by the mobility of holes and electrons in the emitting layer may differ from each other.

The emitting layer can be formed by a known method such as a deposition method, a spin coating method, a LB method (Langmuir Blodgett method) or the like, for example. The emitting layer can also be formed by forming a solution obtained by dissolving a binder such as a resin and material compounds in a solvent into a thin film by a spin coating method and the like.

The emitting layer is preferably a molecular deposited film. The "molecular deposited film" means a thin film formed by deposition of a raw material compound in a vapor phase or a film formed by solidification of a raw material compound in a solution state or a liquid phase state. Normally, this molecular deposited film differs from a thin film (molecular accumulated film) formed by a LB method in aggregation structure or high-order structure, or differ in function derived from such difference in structure.

The content of the emitter materials (dopants), preferably the phosphorescent emitter materials, in the light emitting layer is not particularly limited and selected according to the use of the device, and preferably 0.1 to 70% by mass, and more preferably 1 to 30% by mass. If being 0.1% by mass or more, the amount of light emission is sufficient. If being 70% by mass or less, the concentration quenching can be avoided. The further component in the emitting layer is usually one or more host material, which is preferably present in an amount of 30 to 99.9 % by mass, more preferably 70 to 99% by mass, wherein the sum of the emitter material(s) and the host material(s) is 100% by mass. Suitable host (matrix) and emitter materials are mentioned below. Preferably, at least one host material is a compound of formula (I) according to the invention.

### (1) Phosphorescent emitting layer

The phosphorescent emitting layer usually comprises at least one emitter material and at least one host material. The phosphorescent host is a compound which confines the triplet energy of the phosphorescent dopant efficiently in the light emitting layer to cause the phosphorescent dopant to emit light efficiently.

A host material for phosphorescent emitting layer is usually selected from known phosphorescent host materials. Specific examples of the preferable phosphorescent host are, nitrogen-containing heteroaromatics, such as, indole compounds, carbazole compounds, pyridine compounds, pyrimidine compounds, triazine compounds, quinoline compounds, isoquinoline compounds, quinazoline compounds, nitrogenated-dibenzothiophene compounds, nitrogenated-dibenzofuran compounds, imidazole compounds, such as benzimidazole compounds, imidazopyridine compounds, Benzimidazophenanthridine compounds, benzimidzobenzimidazole compounds; oxygen or sulfur containing heteroaromatics, such as thiophene compounds, furan compounds, benzothiophene compounds, benzofuran compounds, dibenzothiophene compounds, dibenzofuran compounds; aryl or heteroaryl substituted amine compounds; metal complexes; aromatic hydrocarbon compounds, such as benzene compounds naphthalene compounds, phenanthrene compounds, triphenylene compounds, fluorene compounds. Most preferably, the host comprises the composition comprising at least one compound of formula (Ia) or (Ib) and at least one compound of formula (II) according to the present invention.

According to one embodiment, the light-emitting layer comprises at least one emitter material and at least two matrix materials, wherein one of the matrix materials is a compound of the formula (Ia) or (Ib) and the other matrix material(s) is/are used as co-host(s). Suitable host materials other than the compounds of formulae (Ia) and (lb), are (co-hosts) mentioned above, and below (second host material). Most preferably, the light-emitting layer comprises the composition comprising at least one compound of formula (Ia) or (Ib) and at least one compound of formula (II) according to the present invention.

However, it is also possible to use two or more different compounds of formula (Ia) or (Ib) as host material in the light-emitting layer in an OLED of the present application.

Said second host material is selected from general phosphorescent host materials mentioned above. Specific examples are selected from above mentioned compounds, preferably, nitrogen containing heteroaromatics, more preferably, following general formula (N-1). The present invention therefore further relates to an organic electroluminescence device, wherein the light emitting layer comprises a heterocyclic compound represented by the general formula (N-1) and preferably at least one compound of formula (Ia) or (lb). 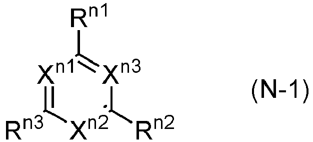
Xⁿ¹ to Xⁿ³ each independently represents CRⁿ⁴ or N,
Rⁿ¹ to Rⁿ⁴ each independently represents hydrogen, halogen atom, a substituted or unsubstituted alkyl group having 1 to 25 carbon atoms, a substituted or unsubstituted alkenyl group having 2 to 25 carbon atoms, a substituted or unsubstituted alkynyl group having 2 to 25 carbon atoms, a substituted or unsubstituted cycloalkyl group having 3 to 25 carbon atoms, a substituted or unsubstituted alkoxy group having 1 to 25 carbon atoms, a substituted or unsubstituted aryl group having 6 to 24 carbon atoms, a substituted or unsubstituted heterocyclic group having 5 to 30 cyclic atoms, a substituted or unsubstituted aryloxy group having 6 to 24 carbon atoms, a substituted or unsubstituted alkylthio group having 1 to 25 carbon atoms, a substituted or unsubstituted arylthio group having 6 to 24 carbon atoms, alkyl or aryl substituted silyl group, alkyl or aryl substituted carbonyl group, or a substituted phosphoryl group,
in the case of at least one of Xⁿ¹ to Xⁿ³ represent CRⁿ⁴, two or more substituents selected among Rⁿ¹ to Rⁿ⁴ may be bonded to each other to form a ring structure.

In one embodiment of the present invention, preferable heteroaromatics for the second host is specific nitrogen containing heteroaromatics with electron donating nitrogen atom(s), such as pyrrole compounds, indole compounds, carbazole compounds, acridine compounds, phenoxadine compounds, phenothiazine compounds, imidazole compounds, benzimidazole compounds, and benzimidazobenzimidazole compounds, which may have additional substituents and additional fused ring structures.

In one embodiment of the present invention, aryl or heteroaryl substituted amine compounds can be preferably used for the second host material. Latter mentioned materials for hole transporting layer can be preferably used as a second host material.

In one embodiment of the present invention, fused aryl compounds or fused heteroaryl compounds are preferable for the second host material.

Most preferably, the second host material is at least one compound of formula (II) according to the present invention.

In the case that the light-emitting layer comprises two host materials, the light-emitting layer is formed from 0.1 to 70% by weight, preferably 1 to 30% by weight, of the at least one emitter material and 30 to 99.9% by weight, preferably 70 to 99% by weight, of a first host and the further matrix material, where the sum total of the at least one emitter material, the further matrix materials adds up to 100% by weight.

The content ratio of the compound of the first host material and the second matrix material as co-host in the light emitting layer is not particularly limited and may be selected accordingly, and the ratio of first host material: second host material is preferably 1:99 to 99:1, more preferably 10:90 to 90:10, most preferably 30:70 to 70:30, each based on the mass of the composition.

A phosphorescent dopant (phosphorescent emitting material) that forms the emitting layer is a compound that can emit light from triplet excited state. The phosphorescent dopant is not limited as long as it can emit from triplet excited state. The phosphorescent dopant is preferably an organic metal complex containing at least one metal selected from Ir, Pt, Os, Au, Cu, Re and Ru and a ligand. It is preferred that the ligand have an ortho-metalated bond. In respect of a high phosphorescent quantum yield and capability of improving external quantum yield of an emitting device, the phosphorescent dopant is preferably a compound having a metal atom selected from Ir, Os and Pt. Further preferable is a metal complex such as an iridium complex, an osmium complex and a platinum complex, with an ortho-metalated complex being more preferable.

The present invention therefore further relates to the organic electroluminescence device according to the present invention, wherein the light emitting layer comprises a phosphorescent material, which is an ortho-metallated complex comprising a metal atom selected from iridium (Ir), osmium (Os) and platinum (Pt).

Among them, an iridium complex and a platinum complex are more preferable, and an ortho-metalated iridium complex is particularly preferable.

The phosphorescent host is a compound having a function of allowing a phosphorescent dopant to emit light efficiently by efficiently confining the triplet energy of the phosphorescent dopant in the emitting layer. The material for an organic EL device according to the invention is preferable as the phosphorescent host. The emitting layer may comprise one kind of the material for an organic EL device according to the invention or may comprise two or more kinds of the material for an organic EL device according to the invention.

When the material for an organic EL device according to the invention is used as a host material of the emitting layer, the emission wavelength of the phosphorescent dopant contained in the emitting layer is not particularly restricted. It is preferred that at least one kind of the phosphorescent dopant materials contained in the emitting layer have a peak of an emission wavelength of 490 nm or more and 700 nm or less, more preferably 490 nm or more and 650 nm or less. As for the emission color of the emitting layer, red, yellow and green are preferable, for example. By using the compound according to the invention as the host material and by forming an emitting layer by doping the phosphorescent dopant having such an emission wavelength, it is possible to obtain a long-lived organic EL device.

In the organic EL device according to the invention, other compounds than the material for an organic EL device according to the invention can appropriately be selected as the phosphorescent host according to the above-mentioned purpose.

The material for an organic EL device according to the invention and other compounds may be used in combination as the phosphorescent host material in the same emitting layer. When plural emitting layers are present, as the phosphorescent host material for one of these emitting layers, the material for an organic EL device according to the invention is used, and as the phosphorescent host material for one of other emitting layers, other compounds than the material for an organic EL device according to the invention may be used. The material for an organic EL device according to the invention can be used in an organic layer other than the emitting layer. In that case, as the phosphorescent host of the emitting layer, other compounds than the material for an organic EL device according to the invention may be used.

Suitable metal complexes (dopants, especially phosphorescent dopants) for use in the inventive OLEDs, preferably as emitter material, are described as following general formula (E-1). 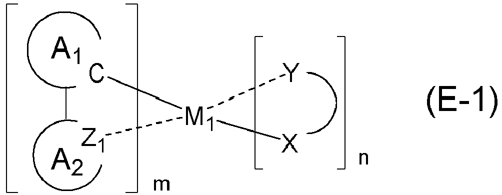
Wherein M₁ is a metal having an atomic weight greater than 40, preferably, Ir, Pt, Pd, Rh, Re, Ru, Os, Tl, Pb, Bi, In, Sn, Sb, Te, Au, or Ag, more preferably Ir, Pt, or Os, most preferably Ir, A₁ represents aryl group having 6 to 24 carbon atoms or heterocyclic group having 3 to 24 cyclic atoms, preferably above mentioned substituents which may have additional substituents,
A₂ represents nitrogen containing heterocyclic group having 3 to 24 cyclic atoms, preferably above mentioned substituents which may have additional substituents,
Z₁ represents C or N, preferably N,
(X-Y) is an ancillary ligand, preferably acetylacetonate compounds, picolinate compounds, more preferably acetylacetonate compounds,
m is a value from 1 to the maximum number of ligands that may be attached to the metal; and m + n is the maximum number of ligands that may be attached to the metal.
If m or n is more than 2, two or more ligands may be the same or different in each occurrence.

According to one embodiment, a metal complex represented by the following general formula (E-2) is more preferable especially for green and yellow emitter, 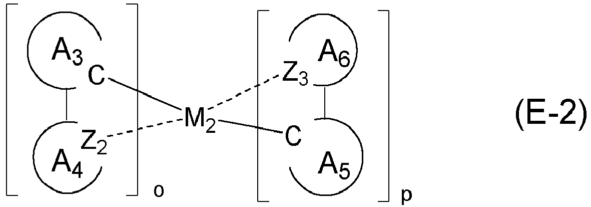
Wherein M₂ is a metal having an atomic weight greater than 40, preferably, Ir, Pt, Pd, Rh, Re, Ru, Os, Tl, Pb, Bi, In, Sn, Sb, Te, Au, or Ag, more preferably Ir, Pt, or Os, most preferably Ir, A₃, A₅ each independently represents aryl group having 6 to 24 carbon atoms or heterocyclic group having 3 to 24 cyclic atoms, preferably above mentioned substituents which may have additional substituents,
A₄, A₆ each independently represents nitrogen containing heterocyclic group having 3 to 24 cyclic atoms, preferably above mentioned substituents which may have additional substituents,
Z₂, Z₃ each independently represents C or N, preferably N,
o is a value from 1 to the maximum number of ligands that may be attached to the metal; and
o + p is the maximum number of ligands that may be attached to the metal.

If o or p is more than 2, two or more ligands may be the same or different in each occurrence.

A metal complex represented by the following general formula (T) or (β) is more preferable. 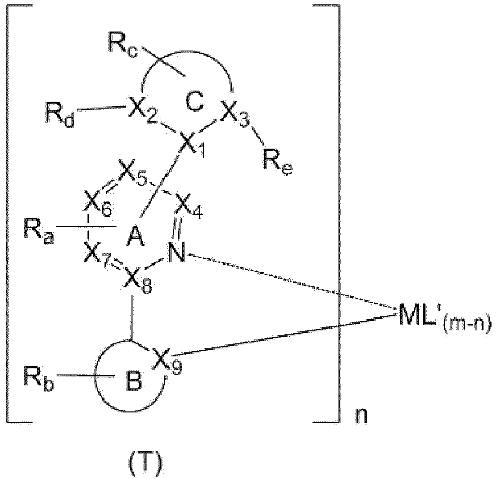
M represents the above mentioned metal atom,
B, C each independently represents aryl group having 6 to 24 carbon atoms or heteroaryl group having 3 to 24 cyclic atoms, preferably phenyl group, dibenzofuran group, dibenzothiophene group, aza-dibenzofuran group, aza-dibenzothiophene group, which may have additional substituents,
A represents a nitrogen containing 6 membered ring structure which may have additional substituents, preferably pyridine, pyrimidine, more preferably pyridine,
X4 to X8 each represents C or N, preferably C,
m represents oxidation state of the metal M, n is 1 or greater than 1,
L' represents following chemical structure, 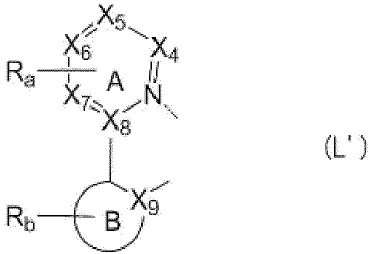
wherein A represents nitrogen containing 6 membered ring structure which may have additional substituents, preferably pyridine, pyrimidine, more preferably pyridine,
B represents aryl group having 6 to 24 carbon atoms or heteroaryl group having 3 to 24 cyclic atoms, preferably phenyl group, dibenzofuran group, dibenzothiophene group, aza-dibenzofuran group, aza-dibenzothiophene group, which may have additional substituents, X9 represents C or N, preferably, N.
Ra, Rb, Rc or Rd each independntly represents hydrogen, a substituted or unsubstituted alkyl group having 1 to 25 carbon atoms, a substituted or unsubstituted alkoxy group having 1 to 25 carbon atoms, a substituted or unsubstituted amino group, a substituted or unsubstituted alkenyl group having 2 to 25 carbon atoms, a substituted or unsubstituted alkynyl group having 2 to 25 carbon atoms, a substituted or unsubstituted aralkyl group having 7 to 24 carbon atoms, a substituted or unsubstituted aryl group having 6 to 24 carbon atoms or a substituted or unsubstituted heterocyclic group having 5 to 30 cyclic atoms, 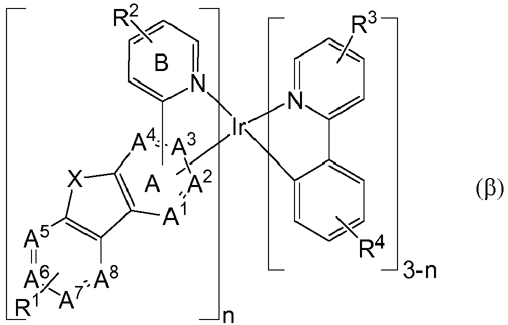
Wherein X represents NR, oxygen atom, sulfur atom, BR or Selenium atom,
R represents hydrogen atom, a substituted or unsubstituted alkyl group having 1 to 25 carbon atoms,
A¹ to A⁸ independently represents CH, CR⁵ or N, preferably CH or CR⁵,
R¹ to R⁵ each independently represents a substituted or unsubstituted alkyl group having 1 to 25 carbon atoms, a substituted or unsubstituted aryl group having 6 to 24 ring carbon atoms, n is 1, 2 or 3, preferably 1.

In another embodiment, a metal complex represented by any one of the following general formula (V), (X), (Y), (Z) can be used. 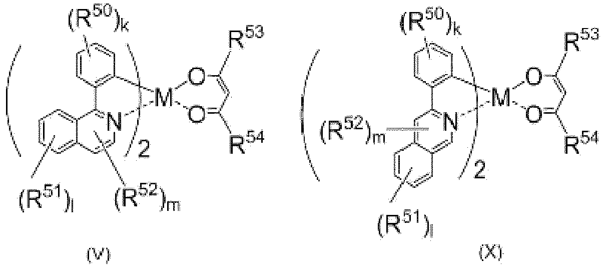 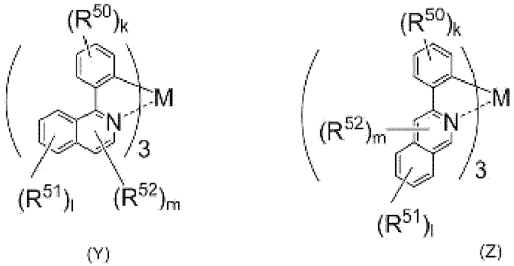
Wherein R⁵⁰ to R⁵² each represents a substituted or unsubstituted alkyl group having 1 to 25 carbon atoms, a substituted or unsubstituted aryl group having 6 to 24 ring carbon atoms,
k is 0, 1, 2, 3 or 4, m is 0, 1 or 2, l is 0, 1, 2, 3 or 4,
M represents iridium atom (Ir), osmium atom (Os) or platinum atom (Pt).

Formula (V) is preferably represented by formula (V-1) or (V-2). Formula (X) is preferably represented by formula (X-1) or (X-2).

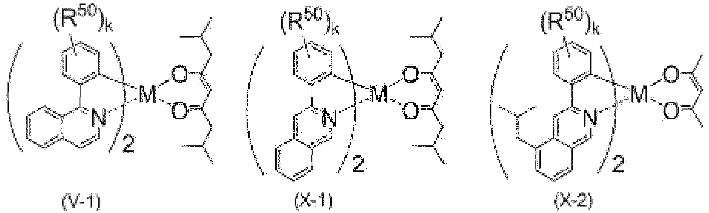

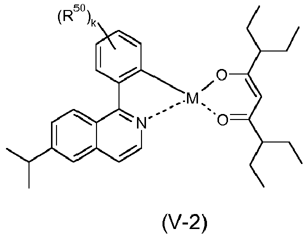

Wherein R⁵⁰, M and k are as defined in formula (V) and (X).

### (2) Fluorescent emitting layer

The fluorescent emitting layer usually comprises at least one emitter material and at least one host material.

A host material for fluorescent emitting layer is usually selected from general host materials, which preferably have wider band-gap than the emitter material to get highly efficient light emission from the emitter through energy transfer mechanism from the excited host to the emitter. Specific examples of the preferable fluorescent host are, substituted or unsubstituted above mentioned heterocyclic compound; or substituted or unsubstituted aromatic hydrocarbon compound, such as oligo-phenylene compounds, naphthalene compounds, fluorene compounds, fluoranthene compounds, anthracene compounds, phenanthrene compounds, pyrene compounds, triphenylene compounds, benzanthracene compounds, chrysene compounds, benzphenanthrene compounds, naphthacene derivstives, benzochrysene compounds, preferably anthracene compounds, pyrene compounds and naphthacene compounds, more preferably, anthracene compounds represented by following general formula (X) especially for fluorescent blue or green device. 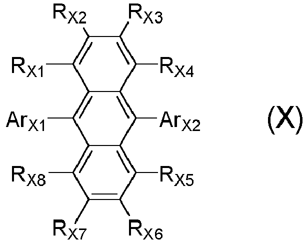
Ar_{X1} and Ar_{X2} are independently a substituted or unsubstituted aryl group including 6 to 50 ring carbon atoms, preferably phenyl group, biphenyl group, naphthyl group, phenanthryl group, fluorenyl group, fluoranthenyl group, anthryl group, pyrenyl group, benzophenanthryl group, triphenylenyl group, benzanthryl group, benzochrysenyl group, or a heterocyclic group including 5 to 50 ring atoms, preferably, benzofuranyl group, benzothiophenyl group, indolyl group, dibenzothiophenyl group, dibenzofuranyl group, carbazolyl group, benzocarbazoryl group, dibenzocarbazoryl group, indolophenanthryl group, naphthobenzofuranyl group, naphthobenzothiophenyl group, dinaphthofuranyl group, dinaphthothiophenyl group, benzophenanthlofuranyl group, benzophenanthlothiophenyl group, benzofurodibenzofuranyl group, benzothiodibenzothiophenyl group, benzofurodibenzotihiophenyl group, benzothiodibenzofuranyl group, more preferably oxygen or sulfur containing heteroaromatics, such as furan or thiophene containing heteroaromatics in one of the part of the heteroaromatics.
R_{X1} to R_{X8} are independently a hydrogen atom, a substituted or unsubstituted aryl group including 6 to 50 ring carbon atoms, a substituted or unsubstituted heterocyclic group including 5 to 50 ring atoms, an alkyl group including 1 to 50 carbon atoms, a substituted or unsubstituted alkoxy group including 1 to 50 carbon atoms, a substituted or unsubstituted aralkyl group including 7 to 50 carbon atoms, a substituted or unsubstituted aryloxy group including 6 to 50 ring carbon atoms, a substituted or unsubstituted arylthio group including 6 to 50 ring carbon atoms, a substituted or unsubstituted alkoxycarbonyl group including 2 to 50 carbon atoms, a substituted or unsubstituted silyl group, a carboxy group, a halogen atom, a cyano group, a nitro group or a hydroxyl group.

An emitter material for fluorescent emitting layer is usually selected from general emitter materials or fluorescent dyes, which preferably have high absorption co-efficiency and high quantum efficiency to get highly efficient light emission from the emitter. Specific examples of the preferable fluorescent emitter are, aromatic hydrocarbon compounds, such as oligo-phenylene compounds, naphthalene compounds, fluorene compounds, fluoranthenyl group, fused fluoranthenyl group, anthracene compounds, phenanthrene compounds, pyrene compounds, triphenylene compounds, benzanthracene compounds, chrysene compounds, benzphenanthrene compounds, naphthacene derivstives, benzochrysene compounds; aromatic or heterocyclic amine compounds represented by following general formula (Y); organic boron compounds represented by general formula (Z), 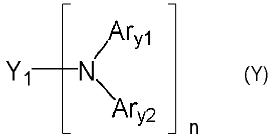 Y₁ is a substituted or unsubstituted aromatic hydrocarbon group including 6 to 50 ring carbon atoms, preferably fused aromatic hydrocarbon group, or substituted or unsubstituted heterocyclic group having 5 to 50 cyclic atoms.

Ar_{y1} and Ar_{y2} are independently a substituted or unsubstituted aryl group including 6 to 50 ring carbon atoms or a substituted or unsubstituted heterocyclic ring group including 5 to 50 ring atoms, preferably, oxygen or sulfur containing heterocyclic group.

Specific examples of Y₁ include the above-mentioned fused aryl group. Y₁ is preferably a substituted or unsubstituted anthryl group; a substituted or unsubstituted pyrenyl group; a substituted or unsubstituted chrysenyl group; substituted or unsubstituted fluorenyl group, especially substituted or unsubstituted mono-, di-, or tri- benzofuro-fused fluorene, or substituted or unsubstituted mono-, di-, or tri- benzothio-fused fluorene; substituted or unsubstituted dibenzofuran containing heterocyclic group; substituted or unsubstituted dibenzothiophene containing heterocyclic group.

n is an integer of 1 to 4, preferably 1 or 2.

### Electron-transporting layer, Electron-injecting layer

The electron-transporting layer is an organic layer that is formed between the emitting layer and the cathode and has a function of transporting electrons from the cathode to the emitting layer. When the electron-transporting layer is formed of plural layers, an organic layer that is nearer to the cathode is often defined as the electron-injecting layer. The electron-injecting layer has a function of injecting electrons from the cathode efficiently to the organic layer unit. The preferred electron-injection materials are alkali metal, alkali metal compounds and alkali metal complexes.

According to one embodiment, it is preferred that an electron-transporting layer further comprises one or more layer(s) like an electron injection layer to enhance efficiency and lifetime of the device, a hole blocking layer, an exciton blocking layer or a triplet blocking layer.

In one embodiment of the present invention, the compound of the formula (I) is present in the electron transporting layer, as an electron transporting material, an electron-injecting material, a hole blocking material, a exciton blocking material and/or a triplet blocking material.

According to one embodiment, it is preferred that an electron-donating dopant be contained in the interfacial region between the cathode and the emitting unit. Due to such a configuration, the organic EL device can have an increased luminance or a long life. Here, the electron-donating dopant means one having a metal with a work function of 3.8 eV or less. As specific examples thereof, at least one selected from an alkali metal, an alkali metal complex, an alkali metal compound, an alkaline earth metal, an alkaline earth metal complex, an alkaline earth metal compound, a rare earth metal, a rare earth metal complex and a rare earth metal compound or the like can be mentioned.

As the alkali metal, Na (work function: 2.36 eV), K (work function: 2.28 eV), Rb (work function: 2.16 eV), Cs (work function: 1.95 eV) and the like can be given. One having a work function of 2.9 eV or less is particularly preferable. Among them, K, Rb and Cs are preferable. Rb or Cs is further preferable. Cs is most preferable. As the alkaline earth metal, Ca (work function: 2.9 eV), Sr (work function: 2.0 eV to 2.5 eV), Ba (work function: 2.52 eV) and the like can be given. One having a work function of 2.9 eV or less is particularly preferable. As the rare-earth metal, Sc, Y, Ce, Tb, Yb and the like can be given. One having a work function of 2.9 eV or less is particularly preferable.

Examples of the alkali metal compound include an alkali oxide such as Li₂O, Cs₂O or K₂O, and an alkali halide such as LiF, NaF, CsF and KF. Among them, LiF, Li₂O and NaF are preferable. Examples of the alkaline earth metal compound include BaO, SrO, CaO, and mixtures thereof such as BaₓSr₁₋ₓO (0<x<1) and BaₓCa₁₋ₓO (0<x<1). Among them, BaO, SrO and CaO are preferable. Examples of the rare earth metal compound include YbF₃, SCF₃, SCO₃, Y₂O₃, Ce₂O₃, GdF₃ and TbF₃. Among these, YbF₃, ScF₃ and TbF₃ are preferable.

The alkali metal complexes, the alkaline earth metal complexes and the rare earth metal complexes are not particularly limited as long as they contain, as a metal ion, at least one of alkali metal ions, alkaline earth metal ions, and rare earth metal ions. Meanwhile, preferred examples of the ligand include, but are not limited to, quinolinol, benzoquinolinol, acridinol, phenanthridinol, hydroxyphenyloxazole, hydroxyphenylthiazole, hydroxydiaryloxadiazole, hydroxydiarylthiadiazole, hydroxyphenylpyridine, hydroxyphenylbenzimidazole, hydroxybenzotriazole, hydroxyfluborane, bipyridyl, phenanthroline, phthalocyanine, porphyrin, cyclopentadiene, β-diketones, and azomethines.

Regarding the addition form of the electron-donating dopant, it is preferred that the electron-donating dopant be formed in a shape of a layer or an island in the interfacial region. A preferred method for the formation is a method in which an organic compound (a light emitting material or an electron-injecting material) for forming the interfacial region is deposited simultaneously with deposition of the electron-donating dopant by a resistant heating deposition method, thereby dispersing the electron-donating dopant in the organic compound. The dispersion concentration of the organic compound: the electron-donating dopant (molar ratio) is 100:1 to 1:100, preferably 5:1 to 1:5.

In a case where the electron-donating dopant is formed into the shape of a layer, the light-emitting material or electron-injecting material which serves as an organic layer in the interface is formed into the shape of a layer. After that, a reductive dopant is solely deposited by the resistant heating deposition method to form a layer preferably having a thickness of from 0.1 nm to 15 nm. In a case where the electron-donating dopant is formed into the shape of an island, the emitting material or the electron-injecting material which serves as an organic layer in the interface is formed into the shape of an island. After that, the electron-donating dopant is solely deposited by the resistant heating deposition method to form an island preferably having a thickness of from 0.05 nm to 1 nm.

The ratio of the main component and the electron-donating dopant in the organic EL device according to the invention is main component: electron-donating dopant = 5 : 1 to 1 : 5 in terms of molar ratio, more preferably 2 : 1 to 1 : 2.

As the electron-transporting material used in the electron-transporting layer other than a compound of the formula (I), an aromatic heterocyclic compound having one or more hetero atoms in the molecule may preferably be used. In particular, a nitrogen containing heterocyclic compound is preferable.

According to one embodiment, it is preferable that the electron-transporting layer comprises a nitrogen containing heterocyclics metal chelate, such as 8-hydroxyquinolinolato aluminum, which is generally called as Alq₃.

According to the other embodiment, it is preferable that the electron-transporting layer comprising substituted or unsubstituted nitrogen containing heterocyclic compound.

Specific examples of the preferable heterocyclic compound for the electron-transporting layer are, 6-membered azine compounds; such as pyridine compounds, pyrimidine compounds, triazine compounds, pyrazine compounds, preferably pyrimidine compounds or triazine compounds; 6-membered fused azine compounds, such as quinolone compounds, isoquinoline compounds, quinoxaline compounds, quinazoline compounds, phenanthroline compounds, benzoquinoline compounds, benzoisoquinoline compounds, dibenzoquinoxaline compounds, preferably quinolone compounds, isoquinoline compounds, phenanthroline compounds; 5-membered heterocyclic compounds, such as imidazole compounds, oxazole compounds, oxadiazole compounds, triazole compounds, thiazole compounds, thiadiazole compounds; fused imidazole compounds, such as benzimidazole compounds, imidazopyridine compounds, naphthoimidazole compounds, benzimidazophenanthridine compounds, benzimidzobenzimidazole compounds, preferably benzimidazole compounds, imidazopyridine compounds or benzimidazophenanthridine compounds.

According to the other embodiment, it is preferable the electron-transporting layer comprises phosphine oxide compound represented as Arₚ₁Arₚ₂Ar_{P3}P=O.

Arₚ₁ to Arₚ₃ are the substituents of phosphor atom and each independently represent substituted or unsubstituted above mentioned aryl group or substituted or unsubstituted above mentioned heterocyclic group.

According to the other embodiment, it is preferable that the electron-transporting layer comprises aromatic hydrocarbon compounds.

Specific examples of the preferable aromatic hydrocarbon compounds for the electron-transporting layer are, oligo-phenylene compounds, naphthalene compounds, fluorene compounds, fluoranthenyl group, anthracene compounds, phenanthrene compounds, pyrene compounds, triphenylene compounds, benzanthracene compounds, chrysene compounds, benzphenanthrene compounds, naphthacene derivstives, and benzochrysene compounds, preferably anthracene compounds, pyrene compounds and fluoranthene compounds.

The present invention therefore relates to an organic electroluminescence device, wherein an electron transporting layer is provided between the cathode and the light emitting layer, and the electron transporting layer comprises at least one compound of formula (I).

The present invention therefore further relates to an organic electroluminescence device, wherein a hole blocking layer is provided between the electron transporting layer and the light emitting layer, and the hole blocking layer comprises at least one compound of formula (Ia) or (lb), or the composition comprising at least one compound of formula (Ia) or (Ib) and at least one compound of formula (II).

According to one embodiment, it is preferred that the other electron transporting region is further comprised between the hole blocking layer comprising the compound of formula (Ia) or (lb), or the composition comprising at least one compound of formula (Ia) or (Ib) and at least one compound of formula (II) and cathode. Said electron transporting region generally comprises one or more electron transporting layer(s).

Above mentioned electron injection materials, such as alkali metal compound or alkali metal complex, preferably comprising as one of electron transporting layer at the interface of cathode. Second electron transporting layer preferably comprises between hole blocking layer and said electron transporting layer comprising electron injection material.

Above mentioned heterocyclic compounds or fused aromatic compounds are preferably used for second electron transporting layer, more preferably heterocyclic compounds represented by general formulae (ET-1), (ET-2), (ET-3) or (ET-4). 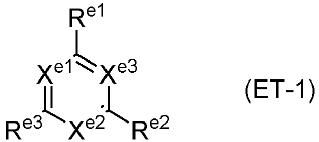
X^{e1} to X^{e3} each independently represents CR^{e4} or N, preferably more than two of X^{e1} to X^{e3} are N,
R^{e1} to R^{e4} each independently represents hydrogen, halogen atom, a substituted or unsubstituted alkyl group having 1 to 25 carbon atoms, a substituted or unsubstituted alkenyl group having 2 to 25 carbon atoms, a substituted or unsubstituted alkynyl group having 2 to 25 carbon atoms, a substituted or unsubstituted cycloalkyl group having 3 to 25 carbon atoms, a substituted or unsubstituted alkoxy group having 1 to 25 carbon atoms, a substituted or unsubstituted aryl group having 6 to 24 carbon atoms, a substituted or unsubstituted heterocyclic group having 5 to 30 cyclic atoms, a substituted or unsubstituted aryloxy group having 6 to 24 carbon atoms, a substituted or unsubstituted alkylthio group having 1 to 25 carbon atoms, a substituted or unsubstituted arylthio group having 6 to 24 carbon atoms, alkyl or aryl substituted silyl group, alkyl or aryl substituted carbonyl group, or a substituted phosphoryl group,
in the case of at least one of X^{e1} to X^{e3} represent CR^{e4}, two or more substituents selected among R^{e1} to R^{e4} may be bonded to each other to form a ring structure. 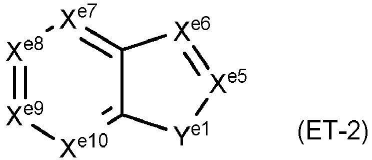
X^{e6} to X^{e10} each independently represents CR^{e5} or N, preferably at least X^{e6} is N,
Y^{e1} represents oxygen atom, sulfur atom, CR^{e6}R^{e7} or NR^{e7},
R^{e5} to R^{e8} each independently represents hydrogen, halogen atom, a substituted or unsubstituted alkyl group having 1 to 25 carbon atoms, a substituted or unsubstituted alkenyl group having 2 to 25 carbon atoms, a substituted or unsubstituted alkynyl group having 2 to 25 carbon atoms, a substituted or unsubstituted cycloalkyl group having 3 to 25 carbon atoms, a substituted or unsubstituted alkoxy group having 1 to 25 carbon atoms, a substituted or unsubstituted aryl group having 6 to 24 carbon atoms, a substituted or unsubstituted heterocyclic group having 5 to 30 cyclic atoms, a substituted or unsubstituted aryloxy group having 6 to 24 carbon atoms, a substituted or unsubstituted alkylthio group having 1 to 25 carbon atoms, a substituted or unsubstituted arylthio group having 6 to 24 carbon atoms, alkyl or aryl substituted silyl group, alkyl or aryl substituted carbonyl group, or a substituted phosphoryl group,
two or more substituents selected among R^{e5} to R^{e8} may be bonded to each other to form a ring structure. 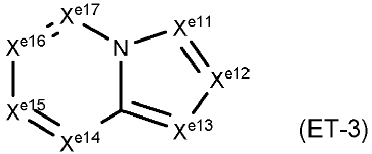
X^{e11} to X^{e17} each independently represents CR^{e9} or N, preferably at least one selected from X^{e11}, X^{e12} and X^{e13} is N,
R^{e9} each independently represents hydrogen, halogen atom, a substituted or unsubstituted alkyl group having 1 to 25 carbon atoms, a substituted or unsubstituted alkenyl group having 2 to 25 carbon atoms, a substituted or unsubstituted alkynyl group having 2 to 25 carbon atoms, a substituted or unsubstituted cycloalkyl group having 3 to 25 carbon atoms, a substituted or unsubstituted alkoxy group having 1 to 25 carbon atoms, a substituted or unsubstituted aryl group having 6 to 24 carbon atoms, a substituted or unsubstituted heterocyclic group having 5 to 30 cyclic atoms, a substituted or unsubstituted aryloxy group having 6 to 24 carbon atoms, a substituted or unsubstituted alkylthio group having 1 to 25 carbon atoms, a substituted or unsubstituted arylthio group having 6 to 24 carbon atoms, alkyl or aryl substituted silyl group, alkyl or aryl substituted carbonyl group, or a substituted phosphoryl group,
two or more substituents selected among R^{e9} may be bonded to each other to form a ring structure. 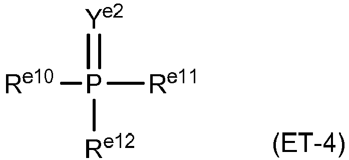
R^{e10} to R^{e12} each independently represents a substituted or unsubstituted aryl group having 6 to 24 carbon atoms, a substituted or unsubstituted heterocyclic group having 5 to 30 cyclic atoms,
two or more substituents selected among R^{e10} to R^{e12} may be bonded to each other to form a ring structure, preferably at least one of R^{e10} to R^{e12} have additional substituted or unsubstituted aryl group having 6 to 24 carbon atoms, or substituted or unsubstituted heterocyclic group having 5 to 30 cyclic atoms,
Y^{e2} represents oxygen atom or sulfur atom.

### Hole-injection layer, Hole-transporting layer

The hole-transporting layer is an organic layer that is formed between the emitting layer and the anode, and has a function of transporting holes from the anode to the emitting layer. If the hole-transporting layer is composed of plural layers, an organic layer that is nearer to the anode may often be defined as the hole-injecting layer. The hole-injecting layer has a function of injecting holes efficiently to the organic layer unit from the anode.

Said hole injection layer is generally used for stabilizing hole injection from anode to hole transporting layer which is generally consist of organic materials.

Organic material having good contact with anode or organic material with p-type doping is preferably used for the hole injection layer.

Acceptor materials, or fused aromatic hydrocarbon materials or fused heterocycles which have high planarity, are preferably used, acceptor materials are more preferably used for the hole injection layer.

Specific examples for acceptor materials are, the quinone compounds with one or more electron withdrawing groups, such as F₄TCNQ(2,3,5,6-tetrafluoro-7,7,8,8-tetracyanoquinodimethane), and 1,2,3-tris[(cyano)(4-cyano-2,3,5,6-tetrafluorophenyl)methylene]cyclopropane; hexa-azatriphenylene compounds with one or more electron withdrawing groups, such as hexa-azatriphenylene-hexanitrile; aromatic hydrocarbon compounds with one or more electron withdrawing groups; and aryl boron compounds with one or more electron withdrawing groups.

p-doping is usually consist of one or more p-dopant materials and one or more matrix materials. Matrix materials preferably have shallower HOMO level and p-dopant preferably have deeper LUMO level to enhance the carrier density of the layer. Aryl or heteroaryl amine compounds are preferably used as the matrix materials. Specific examples for the matrix material are the same as that for hole transporting layer which is explained at the later part. Specific examples for p-dopant are the above mentioned acceptor materials, preferably the quinone compounds with one or more electron withdrawing groups, such as F₄TCNQ, 1,2,3-Tris[(cyano)(4-cyano-2,3,5,6-tetrafluorophenyl)methylene]cyclopropane.

The ratio of the p-type dopant is preferably less than 20% of molar ratio, more preferably less than 10%, such as 1%, 3%, or 5%.

Hole transporting layer is generally used for injecting and transporting holes efficiently, and aromatic or heterocyclic amine compounds are preferably used.

Specific examples for hole transporting layer are represented as general formula (H), 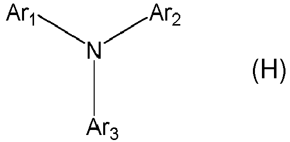 Ar₁ to Ar₃ each independently represents substituted or unsubstituted aryl group having 5 to 50 carbon atoms or substituted or unsubstituted heterocyclic group having 5 to 50 cyclic atoms, preferably phenyl group, biphenyl group, terphenyl group, naphthyl group, phenanthryl group, triphenylenyl group, fluorenyl group, spirobifluorenyl group, indenofluorenyl group, carbazolyl group, dibenzofuranyl group, dibenzothiophenyl group, carbazole substituted aryl group, dibenzofuran substituted aryl group or dibenzothiophene substituted aryl group; two or more substituents selected among Ar¹ to Ar³ may be bonded to each other to form a ring structure, such as a carbazole ring structure, or a acridane ring structure.

According to one embodiment, it is preferable that at least one of Ar₁ to Ar₃ have additional one aryl or heterocyclic amine substituent, more preferably Ar₁ has an additional aryl amino substituent, at the case of that it is preferable that Ar₁ represents substituted or unsubstituted biphenylene group, substituted or unsubstituted fluorenylene group.

A second hole transporting layer is preferably inserted between the first hole transporting layer and the emitting layer to enhance device performance by blocking excess electrons or excitons.

Specific examples for second hole transporting layer is the same as the first hole transporting layer. It is preferably that second hole transporting layer have higher triplet energy to block triplet exciton especially for phosphorescent green device, such as bicarbazole compounds, biphenylamine compounds, triphenylenyl amine compounds, fluorenyl amine compounds, carbazole substituted arylamine compounds, dibenzofuran substituted arylamine compounds, and dibenzothiophene substituted arylamine compounds.

### Spacing layer

The spacing layer is a layer provided between the fluorescent emitting layer and the phosphorescent emitting layer when the fluorescent emitting layer and the phosphorescent emitting layer are stacked in order to prevent diffusion of excitons generated in the phosphorescent emitting layer to the fluorescent emitting layer or in order to adjust the carrier balance. Further, the spacing layer can be provided between the plural phosphorescent emitting layers.

Since the spacing layer is provided between the emitting layers, the material for the spacing layer is preferably a material having both electron-transporting properties and hole-transporting properties. In order to prevent diffusion of the triplet energy in adjacent phosphorescent emitting layers, it is preferred that the spacing layer have a triplet energy of 2.6 eV or more. As the material used for the spacing layer, the same material as those used in the above-mentioned hole-transporting layer can be given.

### Barrier layer

It is preferred that the organic EL device according to the invention have a barrier layer such as an electron-barrier layer, a hole-barrier layer and a triplet barrier layer in a part that is adjacent to the emitting layer. Here, the electron-barrier layer is a layer that serves to prevent leakage of electrons from the emitting layer to the hole-transporting layer, and the hole-barrier layer is a layer that serves to prevent leakage of holes from the emitting layer to the electron-transporting layer.

The triplet barrier layer prevents diffusion of triplet excitons generated in the emitting layer to the surrounding layers, and has a function of preventing energy deactivation of triplet excitons on molecules in the electron-transporting layer other than the emitting dopant by confining the triplet excitons within the emitting layer.

The present invention further relates to an electronic equipment comprising the organic electroluminescence device according to the present invention.

The organic EL device using the inventive compounds of formula (Ia) or (Ib) or the inventive composition comprising at least one compound of formula (Ia) or (Ib) and at least one compound of formula (II) can be used as an emitting device in a panel module used in various displays.

The organic EL device using the inventive compounds of formula (Ia) or (Ib) or the inventive composition comprising at least one compound of formula (Ia) or (Ib) and at least one compound of formula (II) can be used as a display element of a TV, a mobile phone and a PC; or an electronic apparatus such as lightings or the like.

The OLEDs (organic EL devices) can be used in all apparatus in which electroluminescence is useful. Suitable devices (electronic equipment) are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### I Preparation Examples

### Compound 1

### Step 1

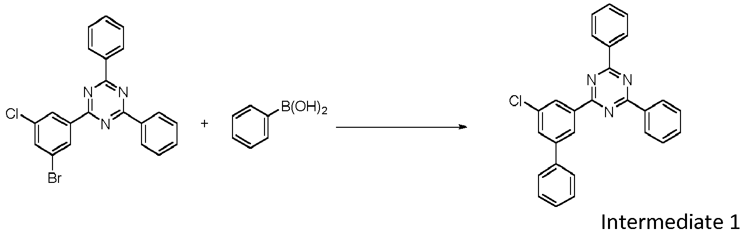

In a nitrogen flushed 2000ml three-necked round-bottomed flask, 2-(3-Bromo-5-chlorophenyl)-4,6-diphenyl-1,3,5-triazine (84.5g, 0.20mol) and phenylboronic acid (26.8g, 0.22mol) were dissolved in toluene (600ml) under nitrogen. 2M sodium carbonate solution (300ml, 0.60mol) and tetrakis(triphenylphosphine)palladium(0) (6.93g, 0.005mol) were added to the reaction mixture. The reaction mixture was heated in an oil bath at 100 °C for 24 hours. After cooling down to room temperature, water was added to the reaction mixture followed by extraction with toluene. The combined organic layers were concentrated. The crude product was added to a silica gel column and was eluted with toluene and hexane to give 55g of white solid (65% yield, Intermediate 1). The identification of the Intermediate 1 was made by FD-MS (field desorption mass spectrometry) analysis.

### Step 2

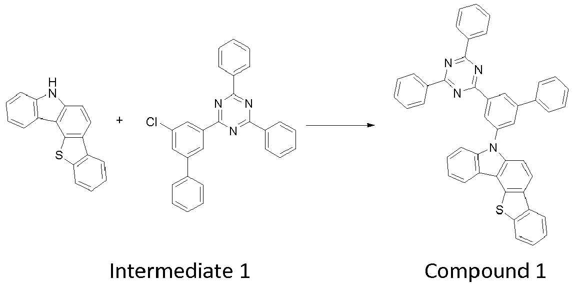

In a nitrogen flushed 1000ml three-necked round-bottomed flask 5*H*-[1]benzothieno[3,2-*c*]carbazole (9.4g, 34mmol), Intermediate 1 (14.4g, 34mmol), 2-dicyclohexylphosphino-2',4', 6'-triisopropylbiphenyl (0.65g, 1.36mmol), tris(dibenzylideneacetone)dipalladium(0) (0.63g, 0.688mmol) and sodium tert-butoxide (4.9g, 50mmol) were dissolved in 240ml xylene under nitrogen. The reaction mixture was heated 135°C with an oil bath for 3 hours. After cooling down to room temperature, 150ml methanol was added to the reaction mixture. The reaction mixture was filtered and precipitate was washed with methanol. The precipitate was added to a silica gel column and was eluted with hot toluene, and then washed with hot xylene twice to give 20g of yellow solid (90% yield, Compound 1). The identification of Compound 1 was made by FD-MS (field desorption mass spectrometry), maximum ultraviolet absorption wavelength (UV(PhMe) λmax) in toluene, and maximum fluorescence wavelength (FL(PhMe, λex=315nm) λmax) in toluene. The results are shown below.

FDMS: calcd. for C45H28N4S=656 , found m/z=656 (M+)
UV(PhMe) λmax: 356nm
FL(PhMe, λex=315nm) λmax: 446nm

### Compound 2

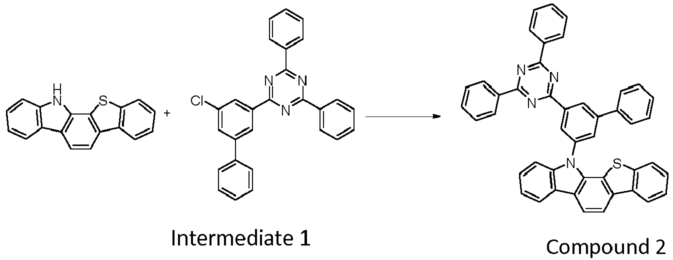

In a nitrogen flushed 1000ml three-necked round-bottomed flask 12*H*-[1]benzothieno[2,3-*a*]carbazole (11.5g, 42mmol), Intermediate 1 (19.2g, 46mmol), 2-dicyclohexylphosphino-2',4', 6'-triisopropylbiphenyl (0.80g, 1.68mmol), tris(dibenzylideneacetone)dipalladium(0) (0.79g, 0.863mmol) and sodium tert-butoxide (6.2g, 65mmol) were dissolved in 300ml xylene under nitrogen. The reaction mixture was heated 135°C with an oil bath for 19 hours. After cooling down to room temperature, 150ml methanol was added to the reaction mixture. The reaction mixture was filtered and precipitate was washed with methanol. The precipitate was washed with hot xylene three times, and then recrystallized from xylene to give 19g of yellow solid (69% yield, Compound 2). The identification of Compound 2 was made by FD-MS (field desorption mass spectrometry), maximum ultraviolet absorption wavelength (UV(PhMe) λmax) in toluene, and maximum fluorescence wavelength (FL(PhMe, λex=322nm) λmax) in toluene. The results are shown below.

FDMS: calcd. for C45H28N4S=656 , found m/z=656 (M+)
UV(PhMe) λmax: 365nm
FL(PhMe, λex=322nm) λmax: 450nm

### II Application Examples 1-2.

A glass substrate with 130 nm-thick indium-tin-oxide (ITO) transparent electrode (manufactured by Geomatec Co., Ltd.) was used as an anode. To eliminate any possible organic residues, the substrate was exposed to N2 plasma for 100s. This treatment also improves the hole injection properties of the ITO. The cleaned substrate was mounted on a substrate holder and loaded into a vacuum chamber. Thereafter, the organic materials specified below were applied by vapor deposition to the ITO substrate at a rate of approx. 0.2-1 Å/sec at about 10⁻⁶ -10⁻⁸ mbar. As a hole injection layer, 5 nm of compound HI was applied. Then 25 nm-thick of compound HT1 was applied as hole transporting layer. Subsequently, a mixture of 10.7% by weight of an emitter PGD 1, a combination of Compound 2 and Compound PH1 as a defined ratio by weight were applied to form a 33 nm-thick phosphorescent-emitting layer. On the emitting layer, a 30 nm-thick layer of compound ET1 was applied as an electron transport layer. Finally, 10 nm-thick mixture of 4% by weight Li metal and 96 % by weight of ET2 was deposited as an electron injection layer and 50 nm-thick Al was then deposited as a cathode to complete the device. The device was sealed with a glass lid and a getter in an inert nitrogen atmosphere with less than 1 ppm of water and oxygen. To characterize the OLED, electroluminescence spectra were recorded at various currents and voltages. 95% lifetime (LT95), the time spent until the initial luminance at 50mA/cm² was reduced to 95%, was recorded. The device results were shown in Table 1.

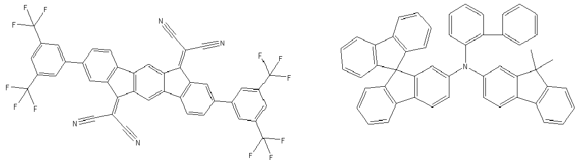

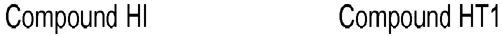

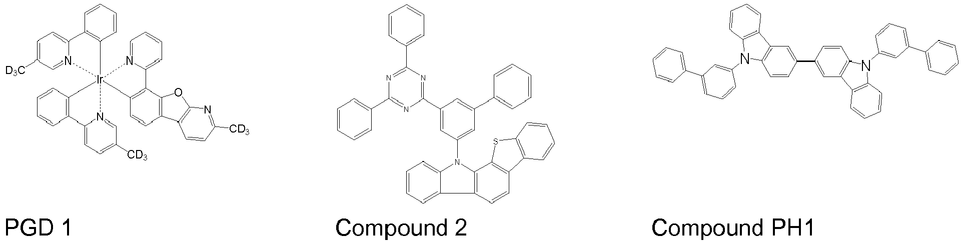

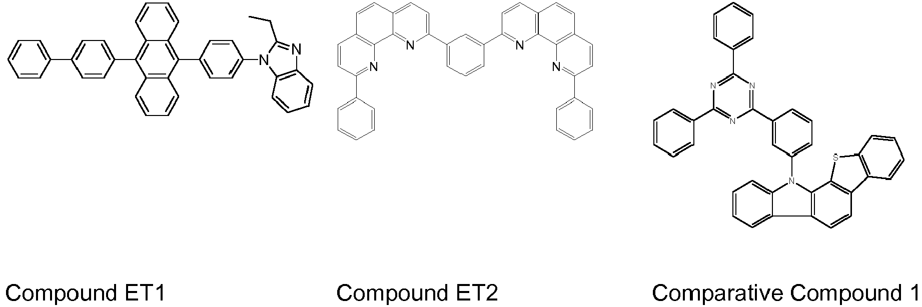

### Comparative Applications Examples 1-2.

Application Example 1 was repeated except for using the Comparative Compound 1 in place of Compound 2. The device results were shown in Table 1.

**Table 1**

| Appl. Ex. | Host 1 | Host 2 | LT95 [hrs] |
|---|---|---|---|
| Appl. Ex. 1 | Compound 2 (26.8 %) | Compound PH1 (62.5%) | 123 |
| Appl. Ex. 2 | Compound 2 (44.5 %) | Compound PH1 (44.5 %) | 149 |
| Comp. Appl. Ex. 1 | Comp. Compound 1 (26.8%) | Compound PH1 (62.5%) | 113 |
| Comp. Appl. Ex. 2 | Comp. Compound 1 (44.5 %) | Compound PH1 (44.5 %) | 108 |

The results shown in Table 1 demonstrated that lifetime was improved in the case when an inventive Compound 2 was used as a green host together with a co-host compound PH1 in an OLED.

## Claims

1. A polycyclic compound represented by formula (Ia) or (Ib): 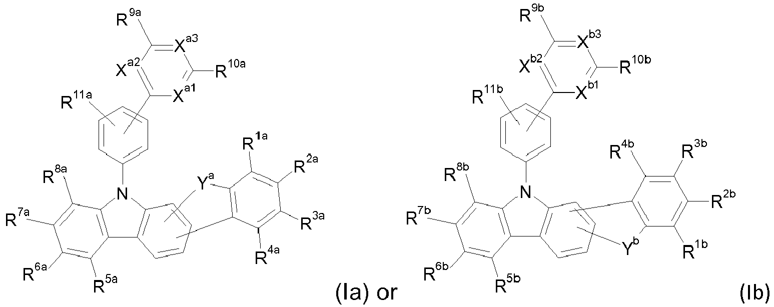 wherein
R^{1a}, R^{2a}, R^{3a}, R^{4a}, R^{5a}, R^{6a}, R^{7a}, R^{8a}, R^{1b}, R^{2b}, R^{3b}, R^{4b}, R^{5b}, R^{6b}, R^{7b} and R^{8b} each independently represents hydrogen, a halogen atom, a substituted or unsubstituted alkyl group having 1 to 25 carbon atoms, a substituted or unsubstituted alkenyl group having 2 to 25 carbon atoms, a substituted or unsubstituted alkynyl group having 2 to 25 carbon atoms, a substituted or unsubstituted cycloalkyl group having 3 to 25 carbon atoms, a substituted or unsubstituted alkoxy group having 1 to 25 carbon atoms, a substituted or unsubstituted aryl group having 6 to 18 carbon atoms, a substituted or unsubstituted heteroaryl group having 5 to 18 ring atoms, a substituted or unsubstituted aryloxy group having 6 to 24 carbon atoms, a substituted or unsubstituted alkylthio group having 1 to 25 carbon atoms, a substituted or unsubstituted arylthio group having 6 to 24 carbon atoms, an alkyl and/or aryl substituted silyl group, an alkyl or aryl substituted carbonyl group, a substituted phosphoryl group or CN;
R^{9a}, R^{10a}, R^{9b} and R^{10b} each independently represents a substituted or unsubstituted aryl group having 6 to 18 carbon atoms or a substituted or unsubstituted heteroaryl group having 5 to 18 ring atoms;
R^{11a} and R^{11b} each independently represents an unsubstituted aryl group having 6 to 18 carbon atoms or an unsubstituted heteroaryl group having 5 to 18 ring atoms;
X^{a1}, X^{a2}, X^{a3}, X^{b1}, X^{b2} and X^{b3} each independently represents N or CR¹², wherein at least two of X^{a1}, X^{a2} and X^{a3} and at least two of X^{b1}, X^{b2} and X^{b3} are N;
R¹² represents in each case independently hydrogen, a substituted or unsubstituted aryl group having 6 to 18 carbon atoms or a substituted or unsubstituted heteroaryl group having 5 to 18 ring atoms;
Y^{a} and Y^{b} each independently represents S or O.

2. The compound according to claim 1, wherein R^{1a}, R^{2a}, R^{3a}, R^{4a}, R^{5a}, R^{6a}, R^{7a}, R^{8a}, R^{1b}, R^{2b}, R^{3b}, R^{4b}, R^{5b}, R^{6b}, R^{7b} and R^{8b} each independently represents hydrogen, a substituted or unsubstituted phenyl group, CN, a substituted or unsubstituted biphenyl group, a substituted or unsubstituted terphenyl group, a substituted or unsubstituted triphenylenyl group, a substituted or unsubstituted naphthyl group, a substituted or unsubstituted phenanthrenyl group, a substituted or unsubstituted dibenzofuranyl group or a substituted or unsubstituted dibenzothiophenyl group.

3. The compound according to claim 2, wherein R^{1a}, R^{2a}, R^{3a}, R^{4a}, R^{5a}, R^{6a}, R^{7a}, R^{8a}, R^{1b}, R^{2b}, R^{3b}, R^{4b}, R^{5b}, R^{6b}, R^{7b} and R^{8b} each represents hydrogen, a substituted or unsubstituted phenyl group or CN.

4. The compound according to any one of claims 1 to 3, wherein R^{9a}, R^{10a}, R^{9b} and R^{10b} each independently represents a substituted or unsubstituted phenyl group, a substituted or unsubstituted biphenyl group, a substituted or unsubstituted terphenyl group, a substituted or unsubstituted triphenylenyl group, a substituted or unsubstituted naphthyl group, a substituted or unsubstituted phenanthrenyl group, a substituted or unsubstituted dibenzofuranyl group or a substituted or unsubstituted dibenzothiophenyl group.

5. The compound according to claim 4, wherein R^{9a}, R^{10a}, R^{9b} and R^{10b} each independently represents an unsubstituted phenyl group, an unsubstituted biphenyl group, an unsubstituted dibenzofuranyl group or an unsubstituted dibenzothiophenyl group.

6. The compound according to any one of claims 1 to 5, wherein R^{11a} and R^{11b} each independently represents an unsubstituted phenyl group, an unsubstituted biphenyl group, an unsubstituted terphenyl group, an unsubstituted triphenylene group, a unsubstituted naphthyl group, a unsubstituted phenanthrenyl group, an unsubstituted dibenzofuranyl group or an unsubstituted dibenzothiophenyl group.

7. The compound according to claim 6, wherein R^{11a} and R^{11b} each independently represents an unsubstituted phenyl group, an unsubstituted biphenyl group, an unsubstituted dibenzofuranyl group or an unsubstituted dibenzothiophenyl group.

8. The compound according to any one of claims 1 to 7, wherein X^{a1}, X^{a2}, X^{a3}, X^{b1}, X^{b2} and X^{b3} each represents N.

9. The compound according to any one of claims 1 to 8, represented by the following formula (Iaa) or (Iba):

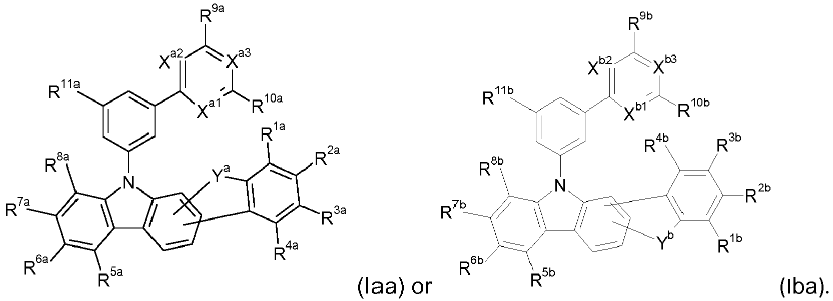

10. A composition comprising
iii) at least one polycyclic compound represented by formula (Ia) or (Ib) as defined in any one of claims 1 to 9, and
iv) at least one compound of formula (II) 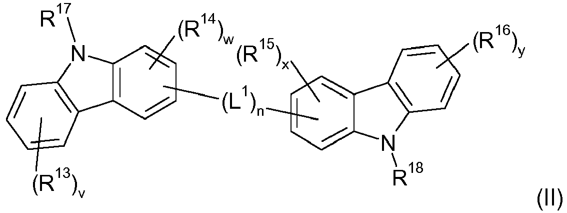 wherein
R¹³, R¹⁴, R¹⁵ and R¹⁶ each independently represents hydrogen, a halogen atom, a substituted or unsubstituted alkyl group having 1 to 25 carbon atoms, a substituted or unsubstituted alkenyl group having 2 to 25 carbon atoms, a substituted or unsubstituted alkynyl group having 2 to 25 carbon atoms, a substituted or unsubstituted cycloalkyl group having 3 to 25 carbon atoms, a substituted or unsubstituted alkoxy group having 1 to 25 carbon atoms, a substituted or unsubstituted aryl group having 6 to 24 carbon atoms, a substituted or unsubstituted heterocyclic group having 5 to 30 ring atoms, a substituted or unsubstituted aryloxy group having 6 to 24 carbon atoms, a substituted or unsubstituted alkylthio group having 1 to 25 carbon atoms, a substituted or unsubstituted arylthio group having 6 to 24 carbon atoms, alkyl and/or aryl substituted silyl group, alkyl and/or aryl substituted carbonyl group, a substituted phosphoryl group, or CN;
v and y are each independently 0, 1, 2, 3 or 4;
w and x are each independently 0, 1, 2 or 3;
R¹⁷ and R¹⁸ are independently a substituted or unsubstituted aromatic hydrocarbon group having 6 to 30 ring carbon atoms or a substituted or unsubstituted heterocyclic group having 5 to 30 ring atoms, an alkyl group having 1 to 25 carbon atoms, a substituted or unsubstituted cycloalkyl group having a ring formed of 3 to 25 carbon atoms, a substituted or unsubstituted aralkyl group having 7 to 24 carbon atoms;
L¹ is a direct bond, a substituted or unsubstituted alkylene group having 1 to 30 carbon atoms, a substituted or unsubstituted cycloalkylene group having a ring structure formed of 3 to 20 carbon atoms, a divalent silyl group having 2 to 20 carbon atoms, a substituted or unsubstituted divalent aromatic hydrocarbon group having a ring structure formed of 6 to 30 carbon atoms, or a substituted or unsubstituted divalent heterocyclic group having a ring structure formed of 5 to 30 atoms;
n is 1, 2 or 3; in the case that n is 2 or 3, L₁ is the same or different in each occurrence.

11. A material for an organic electroluminescence device, comprising at least one compound according to any one of claims 1 to 9 or the composition according to claim 10.

12. An organic electroluminescence device which comprises an organic thin film layer between a cathode and an anode, wherein the organic thin film layer comprises one or more layers and comprises a light emitting layer, and at least one layer of the organic thin film layer comprises at least one compound according to any one of claims 1 to 9 or the composition according to claim 10.

13. The organic electroluminescence device according to claim 12, wherein the light emitting layer comprises at least one compound according to any one of claims 1 to 9 or the composition according to claim 10.

14. The organic electroluminescence device according to claim 11 or claim 12, wherein the light emitting layer comprises a phosphorescent material, which is an ortho-metallated complex comprising a metal atom selected from iridium (Ir), osmium (Os) and platinum (Pt).

15. The organic electroluminescence device according to any one of claims 12 to 14, wherein an electron transporting layer is provided between the cathode and the light emitting layer, and the electron transporting layer comprises at least one compound according to any one of claims 1 to 9 or the composition according to claim 10.

16. The organic electroluminescence device according to any one of claims 12 to 14, wherein a hole blocking layer is provided between the electron transporting layer and the light emitting layer, and the hole blocking layer comprises at least one compound according to any one of claims 1 to 9 or the composition according to claim 10.

17. An electronic equipment comprising the organic electroluminescence device according to any one of claims 12 to 16.

18. A process for preparing a compound of formula (Ia) or (Ib) according to any one of claims 1 to 9, comprising the steps:
(a) Coupling a compound of formula (Ia1) or (Ib1) 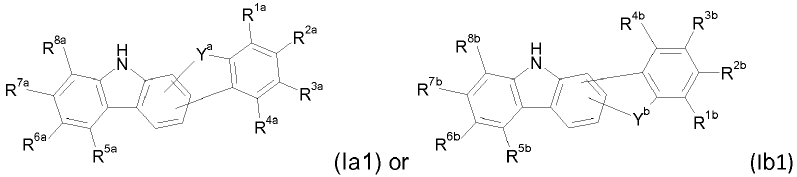 with a compound of formula (IIIa) or (IIIb) 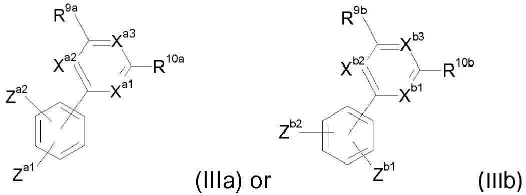 wherein
Z^{a1}, Z^{a2}, Z^{b1} and Z^{b2} each independently represents a halide, preferably selected from the group consisting of I, F, Cl and Br, or a pseudohalide, preferably selected from the group consisting of mesylate, triflate, tosylate and nonaflate, -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring, or -MgX, wherein X is halide,
whereby a compound of formula (Ia2) or (Ib2) is obtained 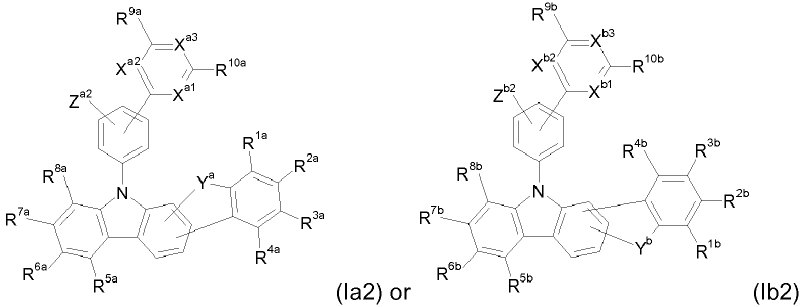
(b) Coupling a compound of formula (Ia2) or (Ib2)
with a compound of formula (IVa) or (IVb)
R^{11a}-Z^{a3} (IVa) or
R^{11b}-Z^{b3} (IVb)
wherein
Z^{a3} and Z^{b3} each independently represents a halide, preferably selected from the group consisting of I, F, Cl and Br, or a pseudohalide, preferably selected from the group consisting of mesylate, triflate, tosylate and nonaflate, -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring, or -MgX, wherein X is halide,
whereby a compound of formula (Ia) or (Ib) is obtained.

19. A process for preparing a compound of formula (Ia) or (Ib) according to any one of claims 1 to 9, comprising the steps:
(a) Coupling a compound of formula (IIIa) or (IIIb) 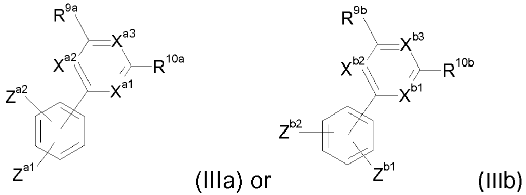 wherein
Z^{a1}, Z^{a2}, Z^{b1} and Z^{b2} each independently represents a halide, preferably selected from the group consisting of I, F, Cl and Br, or a pseudohalide, preferably selected from the group consisting of mesylate, triflate, tosylate and nonaflate, -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring, or -MgX, wherein X is halide,
with a compound of formula (IVa) or (IVb)
R^{11a}-Z^{a3} (IVa) or
R^{11b}-Z^{b3} (IVb)
wherein
Z^{a3} and Z^{b3} each independently represents a halide, preferably selected from the group consisting of I, F, Cl and Br, or a pseudohalide, preferably selected from the group consisting of mesylate, triflate, tosylate and nonaflate, -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring, or -MgX, wherein X is halide,
whereby a compound of formula (Ia3) or (Ib3) is obtained 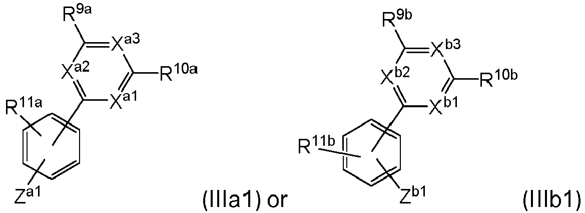
(b) Coupling a compound of formula (IIIa1) or (IIIb1)
with a compound of formula (Ia1) or (Ib1) 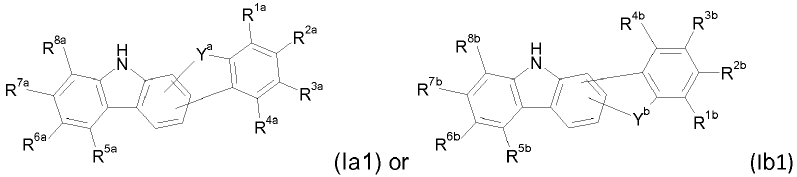 whereby a compound of formula (Ia) or (Ib) is obtained.

20. A process for preparing a compound of formula (Ia) or (Ib) according to any one of claims 1 to 9, comprising the steps:
(a) Coupling a compound of formula (Va) or (Vb) 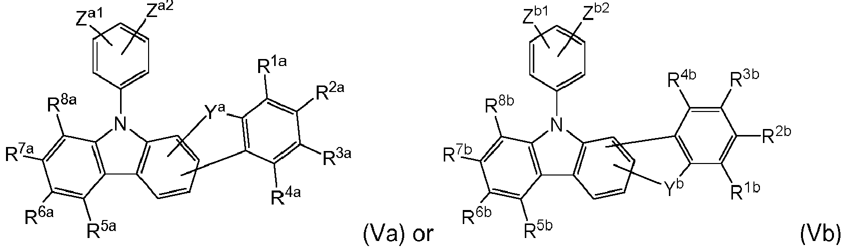 wherein
Z^{a1}, Z^{a2}, Z^{b1} and Z^{b2} each independently represents a halide, preferably selected from the group consisting of I, F, Cl and Br, or a pseudohalide, preferably selected from the group consisting of mesylate, triflate, tosylate and nonaflate; -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring; or -MgX, wherein X is halide,
with a compound of formula (VIa) or (VIb) 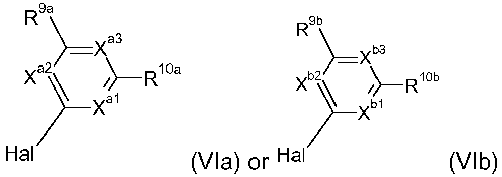
wherein
Hal is a halide, preferably selected from the group consisting of I, F, Cl and Br, or a pseudohalide, preferably selected from the group consisting of mesylate, triflate, tosylate and nonaflate,
whereby a compound of formula (Ia2) or (Ib2) is obtained 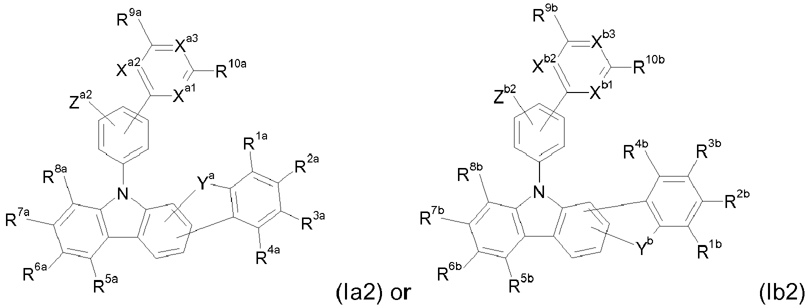
(b) Coupling a compound of formula (Ia2) or (Ib2)
with a compound of formula (IVa) or (IVb)
R^{11a}-Z^{a3} (IVa) or
R^{11b}-Z^{b3} (IVb)
wherein
Z^{a3} and Z^{b3} each independently represents a halide, preferably selected from the group consisting of I, F, Cl and Br, or a pseudohalide, preferably selected from the group consisting of mesylate, triflate, tosylate and nonaflate; -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring; or -MgX, wherein X is halide,
whereby a compound of formula (Ia) or (Ib) is obtained.

21. A process for preparing a compound of formula (Ia) or (Ib) according to any one of claims 1 to 9, comprising the steps:
(a) Coupling a compound of (Va) or (Vb) 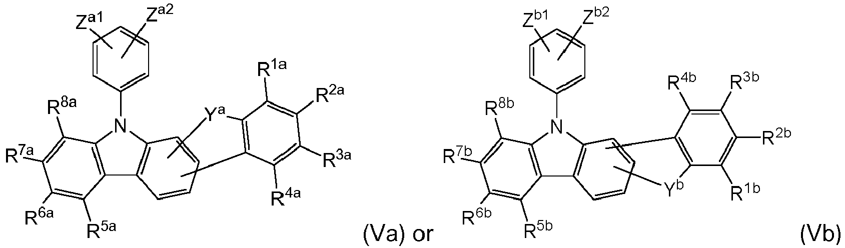 wherein
Z^{a1}, Z^{a2}, Z^{b1} and Z^{b2} each independently represents a halide, preferably selected from the group consisting of I, F, Cl and Br, or a pseudohalide, preferably selected from the group consisting of mesylate, triflate, tosylate and nonaflate; -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring; or -MgX, wherein X is halide,
with a compound of formula (IVa) or (IVb)
R^{11a}-Z^{a3} (IVa) or
R^{11b}-Z^{b3} (IVb)
wherein
Z^{a3} and Z^{b3} each independently represents a halide, preferably selected from the group consisting of I, F, Cl and Br, or a pseudohalide, preferably selected from the group consisting of mesylate, triflate, tosylate and nonaflate; -BQ₂, wherein Q is an unsubstituted alkyl group having 1 to 8 carbon atoms, an unsubstituted cycloalkyl group having 3 to 6 carbon atoms or a cycloalkyl group having 3 to 6 carbon atoms, substituted by one or two unsubstituted alkyl groups having 1 to 8 carbon atoms, a unsubstituted alkoxy group having 1 to 8 carbon atoms, a hydroxyl group, wherein two alkyl groups Q or two alkoxy groups Q together may form a five or six membered ring; or -MgX, wherein X is halide,
whereby a compound of formula (VIIa) or (VIIb) is obtained. 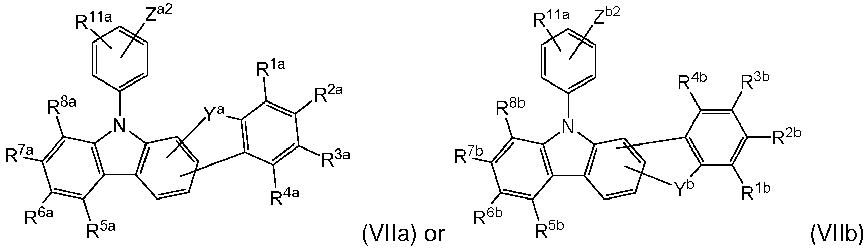
(b) Coupling a compound of formula (VIIa) or (VIIb)
with a compound of formula (VIa) or (VIb) 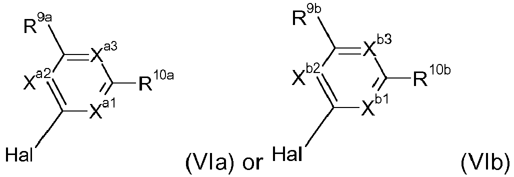 wherein
Hal is a halide, preferably selected from the group consisting of I, F, Cl and Br, or a pseudohalide, preferably selected from the group consisting of mesylate, triflate, tosylate and nonaflate,
whereby a compound of formula (Ia) or (Ib) is obtained.

22. Use of a compound of formula (Ia) or (Ib) according to any one of claims 1 to 9 or a composition according to claim 10 in an organic electroluminescence device.
